# EUROPEAN PATENT SPECIFICATION

(11) **EP 1 135 153 B1**
(45) Date of publication and mention of the grant of the patent: **27.04.2005**
(21) Application number: 99960524.9
(22) Date of filing: 18.11.1999
(51) Int. Cl.: A61K 38/17, A61K 38/19, A61K 39/395, A01K 67/027

(54) **USES FOR EPH RECEPTOR ANTAGONISTS AND AGONISTS TO TREAT VASCULAR DISORDERS**
VERWENDUNG VON EPH-REZEPTOR-ANTAGONISTEN UND AGONISTEN ZUR BEHANDLUNG VON VASKULÄREN KRANKHEITEN
UTILISATIONS RELATIVES AUX ANTAGONISTES ET AGONISTES VIS-A-VIS DU RECEPTEUR DE LA FAMILLE EPH POUR LE TRAITEMENT DES TROUBLES VASCULAIRES

(30) Priority: 20.11.1998 US 109275 P
(43) Date of publication of application: 26.09.2001
(60) Divisional application: 04024047.5
(73) Proprietor: Genentech, Inc., South San Francisco, CA 94080-4990 (US)
(72) Inventor: AGUET, Michel, 1095 Lutry (CH)
(74) Representative: Denison, Christopher Marcus
(86) International application number: PCT/US1999/027534
(87) International publication number: WO 2000/030673

(56) References cited:
- WO-A-97/48804
- WO-A-99/52541
- YANCOPOULOS ET AL: "Vasculogenesis, Angiogenesis, and Growth Factors: Ephrins Enter the Fray at the Border" CELL,US,CELL PRESS, CAMBRIDGE, NA, vol. 93, no. 5, 29 May 1998 (1998-05-29), pages 661-664, XP002120945 ISSN: 0092-8674
- WANG H U ET AL: "Molecular distinction and angiogenic interaction between embryonic arteries and veins revealed by ephrin - B2 and its receptor Eph - B4 [see comments]" CELL,US,CELL PRESS, CAMBRIDGE, NA, vol. 93, no. 5, 29 May 1998 (1998-05-29), pages 741-753, XP002120944 ISSN: 0092-8674
- WANG HAI U ET AL: "Molecular distinction and angiogenic interactions between embryonic arteries and veins revealed by EphrinB2 and its receptor EphB4." 71ST SCIENTIFIC SESSIONS OF THE AMERICAN HEART ASSOCIATION;DALLAS, TEXAS, USA; NOVEMBER 8-11, 1998, vol. 98, no. 17 SUPPL., 27 October 1998 (1998-10-27), page I68 XP000891396 Circulation Oct. 27, 1998 ISSN: 0009-7322

## Description

### BACKGROUND OF THE INVENTION

### Field of the Invention

This invention relates generally to uses for Eph receptor antagonists or agonists. In particular, the present invention provides means for inhibiting or stimulating angiogenesis in a mammal comprising administering to the mammal an effective amount of an Eph receptor antagonist or agonist, respectively.

### Description of Related Art

The formation of new blood vessels either from differentiating endothelial cells during embryonic development (vasculogenesis) or from pre-existing vessels during adult life (angiogenesis) is an essential feature of organ development, reproduction, and wound healing in higher organisms. Folkman and Shing, *J. Biol. Chem*., 267: 10931-10934 (1992); Reynolds *et al., FASEB J.,* 6: 886-892 (1992); Risau *et al., Development*, 102: 471-478 (1988).

Angiogenesis is implicated in the pathogenesis of a variety of disorders. These include solid tumors, intraocular neovascular syndromes such as proliferative retinopathies or age-related macular degeneration (AMD), rheumatoid arthritis, and psoriasis [Folkman *et al. J. Biol. Chem*. 267:10931-10934 (1992); Klagsbrun *et al. Annu. Rev. Physiol*. 53:217-239 (1991); and Garner A, "Vascular diseases". In: *Pathobiology of ocular disease. A dynamic approach*. Garner A, Klintworth GK, Eds. 2nd Edition Marcel Dekker, NY, pp 1625-1710 (1994)]. In the case of solid tumors, the vascularization allows the tumor cells to acquire a growth advantage and proliferative autonomy as compared to the normal cells. Accordingly, a correlation has been observed between density of microvessels in tumor sections and patient survival in breast cancer as well as in several other tumors [Weidner *et al. N Engl J Med* 324:1-6 (1991); Horak *et al. Lancet 340:1120-1124* (1992); and Macchiarini *et al. Lancet* 340:145-146 (1992)].

The search for positive regulators of angiogenesis has yielded many candidates, including aFGF, bFGF, TGF-alpha, TGF-beta, HGF, TNF-alpha, angiogenin, IL-8, etc. (Folkman *et al*. and Kiagsbrun *et al*.). The negative regulators so far identified include thrombospondin [Good *et al. Proc. Natl. Acad. Sci. USA.* 87:6624-6628 (1990)], the 16-kilodalton N-terminal fragment of prolactin [Clapp *et al. Endocrinology,* 133:1292-1299 (1993)], angiostatin [O'Reilly *et al. Cell,* 79:315-328 (1994)] and endostatin [O'Reilly *et al.* Cell, 88:277-285 (1996)].

A number of receptor tyrosine kinases have been described that govern discrete stages of vascular development [Folkman *et al., Cell,* 87:1153-1155 (1996); Hanahan, D., *Science,* 277:48-50 (1997); Risau, W., *Nature,* 386:671-674 (1997); Yancopoulos *et al. Cell,* 93:661-664 (1998)].

Flk-1 (VEGF-R2), a receptor for vascular endothelial growth factor (VEGF), mediates endothelial and hematopoietic precursor cell differentiation [Shalaby *et al., Nature,* 376:62-66 (1995); Carmeliet *et al. Nature,* 380:435-439 (1996); Ferrara *et al. Nature* 380:439-442 (1996)]. VEGF also governs later stages of angiogenesis through ligation of Flt-1 (VEGF-R2) [Fong *et al. Nature*, 376:66-70 (1995)]. Mice that lack Flt-1 had disorganized vascular endothelium with ectopic occurrence of endothelial cells from the earliest stages of vascular development, suggesting that Flt-1 signaling is essential for the proper assembly of endothelial sheets (Fong *et al., supra*).

Another tyrosine kinase receptor, Tie-2 [Dumont *et al. Genes Dev.* 8:1897-1909 (1994); Sato *et al. Nature,* 376:70-74 (1995)] and its ligands Ang-1 [Davis *et al. Cell* 87:1161-1169 (1996); Suri *et al. Cell* 87:1171-1180(1996)] and Ang-2 [Maisonpierre *et al*. Science 277:55-60 (1997)] are involved in assembly of non-endothelial vessel wall components. Tie-1 is involved in maintaining endothelial integrity and its inactivation results in perinatal lethality due to edema and hemorrhage [Sato, *et al. Nature* 376:70-74 (1995)]. The Tie-2 pathway seems to be involved in maturation steps through promoting interactions between the endothelium and surrounding vessel wall components [Suri *et al., supra*; and Vikkula *et al. Cell* 87:1181-1190 (1996)].

The Eph tyrosine kinase subfamily appears to be the largest subfamily of transmembrane receptor tyrosine kinases [Pasquale, E. *Curr. Opin. Cell Biol.* 9:608-615 (1997); and Orioli and Klein, *Trends in Genetics* 13:354-359 (1997)]. Because so many Eph receptor genes have been identified in different species within a short period, each gene has been given multiple names. Accordingly, a unified nomenclature has been developed in which the Eph receptors are divided into two groups on the basis of sequence homologies. Members of the EphA group preferentially bind to Eph ligands with a glycosylphosphatidylinositol (GPI) anchor for membrane anchoring. Such GPI-linked ligands are grouped in the Ephrin-A subclass. Members of the EphB group preferentially bind to ligands possessing a polypeptide transmembrane region for membrane anchoring. Such transmembrane ligands are grouped in the Ephrin-B subclass.

Ephrins and their receptors govern proper cell migration and positioning during neural development, presumably through modulating intercellular repulsion [Pasquale, *supra*; Orioli and Klein, *supra*]. Bidirectional signaling has been observed for some ephrin-B/EphB ligand/receptor pairs [Holland *et al. Nature* 383:722-725 (1996); and Bruckner *et al. Science* 275:1640-1643 (1997)].

The Htk receptor disclosed in Bennett *et al. J. Biol. Chem*. 269:14211-14218 (1994) is a member of the Eph family. This receptor, called "EphB4", is expressed in a variety of tissues including myoepithelial and epithelial cells. Bennett *et al., supra*; Ciossek *et al. Oncogene* 11:2085-2095 (1995); Inada *et al. Blood* 89: 2757-2765 (1997); Nikolova *et al. J. Cell Sci.* 111:2741-2751 (1998). EphB4 receptor expression was recently reported to be estrogen related [Nikolova *et al., supra*].

Ephrin-A1 and Ephrin-B1 have been proposed to have angiogenic properties [Pandey *et al. Science* 268:567-569 (1995); and Stein *et al. Genes Dev.* 12:667-678 (1998)]. Ephrin-B2, a ligand for EphB4 receptor, was recently reported to mark the arterial compartment during early angiogenesis, and mice that lack ephrin-B2 showed severe anomalies in capillary bed formation [Wang *et al. Cell* 93: 741-753 (1998)].

In relation to claims in the present application that are entitled to their claimed priority date, WO99/52541 is prior art for the purposes of novelty only, and only to the extent that it is entitled to one of its own claimed priority dates. WO99/52541 discloses that antagonists of EphB4 will inhibit angiogenesis; a separate passage indicates that the invention relates to methods of enhancing or inhibiting angiogenesis (including angiogenesis in tumors). While each of the three priority documents refers both (1) to the inhibition of angiogenesis and (2) to the inhibition of tumors, none contains a passage that directly and unambiguously links the inhibition of angiogenesis using antagonists of an Eph receptor with the treatment of tumors.

### SUMMARY OF THE INVENTION

The present invention relates to means for inhibiting angiogenesis or treating a vascular anomaly in a mammal comprising administering to the mammal an amount of an Eph receptor antagonist which is effective for inhibiting angiogenesis or for treating the vascular anomaly in the mammal. Thus, the present invention provides means for treating diseases or disorders characterized by undesirable or excessive vascularization, as defined in the claims including by way of example tumors, and especially solid malignant tumors, rheumatoid arthritis, psoriasis, atherosclerosis, diabetic and other retinopathies, retrolental fibroplasia, age-related macular degeneration, ncovascular glaucoma, hemangiomas, thyroid hyperplasias (including Grave's disease), corneal and other tissue transplantation, and chronic inflammation, by administering an effective amount of an Eph receptor antagonist to a patient in need thereof. The invention further provides means for treating diseases or disorders characterized by undesirable or excessive vascular permeability, as defined in the claims, such as edema associated with brain tumors, ascites associated with malignancies, Meigs' syndrome, lung inflammation, nephrotic syndrome, pericardial effusion (such as that associated with pericarditis), and pleural effusion.

In one embodiment, the Eph receptor antagonist is means for treating a vascular anomaly (*e*.*g*. small vessel anomalies) resulting from estrogen therapy. Administration of the Eph receptor antagonist may precede or follow administration of estrogen to the patient. Concomitant therapy is also contemplated, for example, where the Eph receptor antagonist and estrogen are provided in the same composition.

In another aspect, the invention provides means for treating cancer, such as a solid malignant tumor, in a mammal comprising administering to the mammal a therapeutically effective amount of an Eph receptor antagonist.

In a further embodiment, the invention pertains to means for treating vascular tissue or stimulating angiogenesis in a mammal comprising administering to the mammal an amount of an Eph receptor agonist which is effective for treating the vascular tissue or for stimulating angiogenesis. In another embodiment, the invention provides a method of treating a trauma to the vascular network in a mammal comprising administering to a mammal suffering from the trauma a therapeutically effective amount of an Eph receptor agonist. The trauma is, for example, diabetic ulcers or a wound of the blood vessels or heart.

### Brief Description of the Drawings

Figure I depicts schematically murine EphB4 which comprises 987 amino acids and contains a signal peptide, a globulin-like domain, a cysteine rich region (aa184-373), two fibronectin III repeats (FNIII, aa 324-408, 434-509), and a cytoplasmic tyrosine kinase domain (TK, aa 615-878) which are all conserved within the Eph family. The genomic organization of the murine EphB4 receptor gene was determined only in part. Black boxes: known exons; open boxes, not mapped.
Figure 2 depicts Southern blot analysis of EcoRI digested genomic DNA derived from E9.5 embryos. Probe: 1.5 kb XbaI-EcoRI fragment (flanking probe) derived from genomic sequences downstream of the targeting vector (see Fig. 1).
Figure 3A shows a whole mount *in situ* hybridization of E9.5.
Figure 3B is a dark field representation of E9.5 EphB4+/- (left) and EphB4-/- (right) embryos.
Figures 4A-E show histological and whole mount analysis of anti-PECAM immunostained E9.5 embryos. Figure 4A shows a coronal head section of E9.5 embryos (x240). Figure 4B shows a section of anterior limb buds of E9.5 embryos (x960). Figure 4C shows coronal trunk section of E9.5 embryos (x240) (da, dorsal aorta; cv, cardinal vein). Figure 4D shows whole mount anti-PECAM stained E9.5 embryos (da, dorsal aorta; aa, aortic arch). Figure 4E shows magnification of anterior collecting head veins (acv) upstream of the anterior cardinal vein.
Figure 5A shows sections of anti-PECAM stained E10.0 yolk sac (x 120).
Figure 5B shows sections of anti-PECAM stained E9.5 yolk sac (x 480).
Figure 6 shows coronal head section of E9.5 embryos: enlargement of neuroepithelium (x960).

### Detailed Description of the Preferred Embodiments

### I. Definitions

The term "Eph receptor" refers to a tyrosine kinase receptor which belongs to the Eph family of receptors. The Eph family of receptors is reviewed, for example, in Pasquale, E. *Curr. Opin. Cell Biol.* 9:608-615 (1997); and Orioli and Klein, *Trends in Genetics* 13:354-359 (1997). The Eph family comprises fourteen structurally related transmembrane receptor tyrosine kinases, each having an extracellular region comprising a series of modules; a putative immunoglobulin (Ig) domain at the amino terminus, followed by a cysteine-rich region and two fibronectin type III repeats near the single membrane-spanning segment, a cytoplasmic region comprising a highly conserved tyrosine kinase domain flanked by a juxtamembrane region and a carboxyl-terminal tail, which are less conserved. The various variant forms with deletions, truncations, substitutions, or insertions are included in the present definition. Moreover, the Eph receptor, Mep, which lacks kinase activity and other Eph receptor molecules to be discovered are included in the present definition. The Eph receptors can be divided into two groups on the basis of sequence homologies.

Eph receptors of the EphA group, designated "EphA receptors" herein, preferentially interact with glycosylphosphatidylinositol (GPI)-linked ligands (of the Ephrin-A subclass, which presently comprises five ligands). Specific EphA receptors include: EphA1 (also called Eph and Esk); EphA2 (also called Eck, mEck, Myk2, Sek2); EphA3 (also termed Hek, Mek4, Tyro4 and Cek4); EphA4 (also known as Hek8, Sek1, Tyro1, and Cek8); EphA5 (also called Hek7, Bsk, Ehk1, Rek7 and Cek7); EphA6 (also called mEhk2 and Ehk2); EphA7 (otherwise named Hek11, Mdk1, Ebk, Ehk3); and Eph8 (also termed Eek and mEek) and naturally occurring variants thereof.

Eph receptors of the EphB group, designated "EphB receptors" herein, preferentially interact with transmembrane ligands (of the Ephrin-B subclass, which presently comprises three ligands). Specific EphB receptors include EphB I (otherwise named Net, Elk and Cek6); EphB2 (other names include Erk, Hek5, Drt, Nuk, Sek3, Tyro5, Cek5 and Qek5); EphB3 (also called Hek2, Sek4, Mdk5, Tyro6, Cek10 and Qek10); EphB4 (also designated Htk, HpTK5, Myk1, Mdk2 and Tyro 11); EphB5 (also called Cek9); and EphB6 (Hep and Mep are other names for this receptor) and naturally occurring variants thereof.

The preferred Eph receptor is a "native sequence" Eph receptor (see definition below). Preferably, the Eph receptor is "human Eph receptor"; *i.e.* having the amino acid sequence of an Eph receptor from a human source.

A "native sequence" polypeptide is one which has the same amino acid sequence as a polypeptide (*e.g.,* Eph receptor or Eph ligand) derived from nature. Such native sequence polypeptides can be endogenous (*i*.*e*. present *in vivo* in a mammal), isolated from nature, or can be produced by recombinant or synthetic means. Thus, a native sequence polypeptide can have the amino acid sequence of naturally occurring human polypeptide, murine polypeptide, or polypeptide from any other mammalian species.

One preferred Eph receptor herein is "EphB4 receptor" (*i*.*e*. Htk receptor) such as a human Eph receptor comprising an amino acid sequence as in Bennett *et al. J. Biol. Chem*. 269(19):14211-14218 (1994), expressly incorporated herein by reference.

The term "Eph ligand" herein refers to a polypeptide which binds to and, optionally, activates *(e.g.* stimulates tyrosine phosphorylation of) an Eph receptor.

The Eph ligand may be a "GPI-linked Eph ligand", *i*.*e*. comprising a glycosylphosphatidylinositol or GPI anchor. Exemplary GPI-linked Eph ligands include Ephrin-A1 (also called Lerk1 and B61); Ephrin-A2 (otherwise known as Elf1 and Cek7-L); Ephrin-A3 (also termed Lerk3 and Ehkl-L); Ephrin-A4 (also called Lerk4); and Ephrin-A5 (other names are Lerk7, All and Rags) and naturally occurring variants thereof.

Alternatively, the Eph ligand may be a "transmembrane Eph ligand", *i*.*e*. comprising a transmembrane polypeptide domain and preferably further comprising a cytoplasmic polypeptide domain. Examples of transmembrane Eph ligands include Ephrin-B1 (also called Lerk2, Elk-L and Cek5-L); Ephrin-B2 (otherwise known as Lerk5, Htk-L and Elf-2); and Ephrin-B3 (also designated Nlerk2 and Elk-L3) and naturally occurring variants thereof.

The preferred Eph ligand is a transmembrane Eph ligand, and most preferred is Ephrin-B2 (*i*.*e*. Htk ligand) comprising, for example, an amino acid sequence as in GenBank accession nos. L38847 (murine Htk ligand) or L38734 (human Htk ligand) [see, also, Bennett *et al. PNAS (USA)* 92:1866-1870 (1995), expressly incorporated herein by reference].

The term "amino acid sequence variant" refers to polypeptides having amino acid sequences that differ to some extent from a native sequence polypeptide. Ordinarily, amino acid sequence variants will possess at least about 70% homology with at least one receptor binding domain of a native sequence Eph ligand or with at least one ligand binding domain of a native sequence Eph receptor, and preferably, they will be at least about 80%, more preferably at least about 90% homologous with such receptor or ligand binding domains. The amino acid sequence variants possess substitutions, deletions, and/or insertions at certain positions within the amino acid sequence of the native amino acid sequence.

"Homology" is defined as the percentage of residues in the amino acid sequence variant that are identical after aligning the sequences and introducing gaps, if necessary, to achieve the maximum percent homology. Methods and computer programs for the alignment are well known in the art. One such computer program is "Align 2", authored by Genentech, Inc., which was filed with user documentation in the United States Copyright Office, Washington, DC 20559, on December 10, 1991.

The term "receptor binding domain" is used herein to designate any region of an Eph ligand retaining at least a qualitative receptor binding ability of a corresponding native sequence Eph ligand. Amino acid sequence variants and/or derivatives of a native sequence-receptor-binding domain are encompassed in this definition.

The term "ligand binding domain" as used herein refers to any region of an Eph receptor retaining at least a qualitative ligand binding activity of a corresponding native sequence Eph receptor. Amino acid sequence variants and/or derivatives of a native sequence ligand binding domain are encompassed in this definition.

The term "soluble Eph receptor" or "soluble Eph ligand" herein refers to a form of the Eph receptor or Eph ligand which is essentially free of a membrane anchoring region of the native molecule or a form which has an inactivated membrane anchoring region. By "membrane anchoring region" is meant a transmembrane domain (and optionally a cytoplasmic domain) of an Eph receptor or Eph ligand, or a GPI anchor of an Eph ligand. By "essentially free" is meant that the molecule has less than 1% of such membrane anchoring region, preferably 0.5 to 0% of such region, and more preferably 0.1 to 0% of such region.

The term "antagonist" when used herein refers to a molecule which is capable of inhibiting one or more of the biological activities of a target molecule, such as an Eph receptor. Antagonists may act by interfering with the binding of a receptor to a ligand and vice versa, by incapacitating or killing cells which have been activated by a ligand, and/or by interfering with receptor or ligand activation (e.g. tyrosine kinase activation) or signal transduction after ligand binding to a cellular receptor. The antagonist may completely block receptor-ligand interactions or may substantially reduce such interactions. All such points of intervention by an antagonist shall be considered equivalent for purposes of this invention. Thus, included within the scope of the invention are antagonists (*e*.*g*. neutralizing antibodies) that bind to an Eph receptor, Eph ligand or a complex of an Eph receptor and Eph ligand; amino acid sequence variants or derivatives of an Eph receptor or Eph ligand which antagonize the interaction between an Eph receptor and Eph ligand; soluble Eph receptor or soluble Eph ligand, optionally fused to a heterologous molecule such as an immunoglobul in region (*e.g.* an immunoadhesin); a complex comprising an Eph receptor in association with Eph ligand; synthetic or native sequence peptides which bind to Eph receptor or Eph ligand; small molecule antagonists; and nucleic acid antagonists (*e*.*g*. antisense).

An "agonist" herein is a molecule which is capable of activating one or more of the biological activities of a target molecule, such as an Eph receptor. Agonists may, for example, act by activating a target molecule and/or mediating signal transduction. Included within the scope of the invention are Eph receptor or Eph ligand themselves; agonists (*e*.*g*. agonist antibodies) that bind to an Eph receptor, Eph ligand or a complex of an Eph receptor and Eph ligand; amino acid sequence variants or derivatives of an Eph receptor or Eph ligand which activate the Eph receptor or Eph ligand (*e*.*g*. a bivalent molecule such as an immunoadhesin which activates the Eph receptor or ligand); synthetic or native sequence peptides which bind to and activate an Eph receptor or Eph ligand; small molecule agonists; and a gene encoding Eph receptor or Eph ligand (*i*.*e*. for gene therapy).

"Treatment" refers to both therapeutic treatment and prophylactic or preventative measures. Those in need of treatment include those already with the disorder as well as those in which the disorder is to be prevented.

"Mammal" for purposes of treatment refers to any animal classified as a mammal, including humans, domestic and farm animals, and zoo, sports, or pet animals, such as dogs, horses, cats, cows, etc. Preferably, the mammal is human. The human to be treated herein includes an embryo or fetus (*i.e.* wherein the mammal is treated in the uterus), infant, child, pubescent adult or adult.

"Effective amount" or "therapeutically effective amount" of the agonist or antagonist is an amount that is effective either to prevent, lessen the worsening of, alleviate, or cure the treated condition.

A "therapeutically effective amount", in reference to the treatment of cancer refers to an amount capable of invoking one or more of the following effects: (1) inhibition, to some extent, of tumor growth, including, slowing down and complete growth arrest; (2) reduction in the number of tumor cells; (3) reduction in tumor size; (4) inhibition (i.e., reduction, slowing down or complete stopping) of tumor cell infiltration into peripheral organs; (5) inhibition (*i*.*e*., reduction, slowing down or complete stopping) of metastasis; (6) enhancement of anti-tumor immune response, which may, but does not have to, result in the regression or rejection of the tumor; and/or (7) relief, to some extent, of one or more symptoms associated with the disorder.

The term "inhibiting angiogenesis" refers to the act of substantially preventing or reducing the development of blood vessels in a treated mammal.

The expressions "stimulating angiogenesis" or "promoting angiogenesis" refer to the act of substantially increasing the development of blood vessels in a treated mammal.

"Diseases or disorders characterized by undesirable or excessive vascularization" include, by way of example, tumors, and especially solid malignant tumors, rheumatoid arthritis, psoriasis, atherosclerosis, diabetic and other retinopathies, retrolental fibroplasia, age-related macular degeneration, neovascular glaucoma, hemangiomas, thyroid hyperplasias (including Grave's disease), corneal and other tissue transplantation, and chronic inflammation.

Examples of "diseases or disorders characterized by undesirable or excessive vascular permeability" include edema associated with brain tumors, ascites associated with malignancies, Meigs' syndrome, lung inflammation, nephrotic syndrome, pericardial effusion (such as that associated with pericarditis), and pleural effusion.

The expression "trauma to the vascular network" refers to trauma, such as injuries, to the blood vessels or heart, including the vascular network of organs, to which a mammal is subjected. Examples of such trauma include wounds, incisions, and ulcers, *e.g.*, diabetic ulcers and wounds or lacerations of the blood vessels or heart. Trauma includes conditions caused by internal events as well as those that are imposed by an extrinsic agent such as a pathogen, which can be improved by promotion of angiogenesis. It also refers to the treatment of wounds in which vascularization or re-endothelialization is required for healing.

The term "antibody" herein is used in the broadest sense and specifically covers monoclonal antibodies (including full length monoclonal antibodies), polyclonal antibodies, multispecific antibodies (*e.g.*, bispecific antibodies), and antibody fragments. "Antibody fragments" comprise a portion of a full length antibody, generally the antigen binding region or variable domains thereof. Examples of antibody fragments include Fab, Fab', F(ab')₂, and Fv fragments; diabodies; linear antibodies; single-chain antibody molecules; and multispecific antibodies formed from antibody fragments.

The term "monoclonal antibody" as used herein refers to an antibody obtained from a population of substantially homogeneous antibodies, *i.e.*, the individual antibodies comprising the population are identical except for possible naturally occurring mutations that may be present in minor amounts. Monoclonal antibodies are highly specific, being directed against a single antigenic site. Furthermore, in contrast to conventional (polyclonal) antibody preparations which typically include different antibodies directed against different determinants (epitopes), each monoclonal antibody is directed against a single determinant on the antigen. The modifier "monoclonal" indicates the character of the antibody as being obtained from a substantially homogeneous population of antibodies, and is not to be construed as requiring production of the antibody by any particular method. For example, the monoclonal antibodies to be used in accordance with the present invention may be made by the hybridoma method first described by Kohler *et al., Nature* 256:495 (1975), or may be made by recombinant DNA methods (see, *e*.*g*., U.S. Patent No. 4,816,567). The "monoclonal antibodies" may also be isolated from phage antibody libraries using the techniques described in Clackson *et al., Nature* 352:624-628 (1991) and Marks *et al., J. Mol. Biol*. 222:581-597 ( 1991 ), for example.

The monoclonal antibodies herein specifically include "chimeric" antibodies (immunoglobulins) in which a portion of the heavy and/or light chain is identical with or homologous to corresponding sequences in antibodies derived from a particular species or belonging to a particular antibody class or subclass, while the remainder of the chain(s) is identical with or homologous to corresponding sequences in antibodies derived from another species or belonging to another antibody class or subclass, as well as fragments of such antibodies [U.S. Patent No. 4,816,567; and Morrison *et al., Proc. Natl. Acad. Sci. USA* 81:6851-6855 (1984)].

"Humanized" forms of non-human (*e.g.*, murine) antibodies are chimeric antibodies which contain minimal sequence derived from non-human immunoglobulin. For the most part, humanized antibodies are human immunoglobulins (recipient antibody) in which hypervariable region residues of the recipient are replaced by hypervariable region residues from a non-human species (donor antibody) such as mouse, rat, rabbit or non-human primate having the desired specificity, affinity, and capacity. In some instances, Framework Region (FR) residues of the human immunoglobulin are replaced by corresponding non-human residues. Furthermore, humanized antibodies may comprise residues which are not found in the recipient antibody or in the donor antibody. These modifications are made to further refine antibody performance. In general, the humanized antibody will comprise substantially all of at least one, and typically two, variable domains, in which all or substantially all of the hypervariable regions correspond to those of a non-human immunoglobulin and all or substantially all of the FRs are those of a human immunoglobulin sequence. The humanized antibody optionally also will comprise at least a portion of an immunoglobulin constant region (Fc), typically that of a human immunoglobulin. For further details, see Jones *et al., Nature* 321:522-525 (1986); Reichmann *et al., Nature* 332:323-329 (1988); and Presta, *Curr. Op. Struct. Biol*. 2:593-596 (1992).

The term "hypervariable region" when used herein refers to the amino acid residues of an antibody which are responsible for antigen binding. The hypervariable region comprises amino acid residues from a "Complementarity Determining Region" or "CDR" [i.e. residues 24-34 (L1), 50-56 (L2) and 89-97 (L3) in the light chain variable domain and 31-35 (H1), 50-65 (H2) and 95-102 (H3) in the heavy chain variable domain; *Kabat et al., Sequences of Proteins of Immunological Interest*, 5th Ed. Public Health Service, National Institutes of Health, Bethesda, MD. (1991)] and/or those residues from a "hypervariable loop" [i.e. residues 26-32 (L1), 50-52 (L2) and 91-96 (L3) in the light chain variable domain and 26-32 (H1), 53-55 (H2) and 96-101 (H3) in the heavy chain variable domain; Chothia and Lesk *J. Mol. Biol*. 196:901-917 (1987)]. "Framework Region" or "FR" residues are those variable domain residues other than the hypervariable region residues as herein defined.

"Single-chain Fv" or "sFv" antibody fragments comprise the VH and VL domains of antibody, wherein these domains are present in a single polypeptide chain. Generally, the Fv polypeptide further comprises a polypeptide linker between the VH and VL domains which enables the sFv to form the desired structure for antigen binding. For a review of sFv see Pluckthun in *The Pharmacology of Monoclonal Antibodies,* vol. 113, Rosenburg and Moore eds. Springer-Verlag, New York, pp. 269-315 (1994).

The term "diabodies" refers to small antibody fragments with two antigen-binding sites, which fragments comprise a heavy chain variable domain (VH) connected to a light chain variable domain (VL) in the same polypeptide chain (VH - VL). By using a linker that is too short to allow pairing between the two domains on the same chain, the domains are forced to pair with the complementary domains of another chain and create two antigen-binding sites. Diabodies are described more fully in, for example, EP 404,097; WO 93/11161; and Hollinger *et al., Proc. Natl. Acad. Sci. USA* 90:6444-6448 (1993).

The expression "linear antibodies" when used throughout this application refers to the L-F(ab')₂ antibodies described in US Patent 5,641,870 issued June 24, 1997. Briefly, these antibodies comprise a pair of tandem Fd segments (VH-CH1-VH-CH1) which, when combined with two light chains, form a pair of antigen binding regions. Linear antibodies can be bispecific or monospecific.

As used herein, the term "immunoadhesin" designates antibody-like molecules which combine the "binding domain" of a heterologous "adhesin" protein (*e*.*g*. an Eph receptor or Eph ligand) with an immunoglobulin constant domain amino acid sequence. Structurally, the immunoadhesins comprise a fusion of the adhesin amino acid sequence with the desired binding specificity which is other than the antigen recognition and binding site (antigen combining site) of an antibody (*i*.*e*. is "heterologous") and an immunoglobulin constant domain sequence.

An "antibody-immunoadhesin chimera" comprises a molecule which combines at least one binding domain of an antibody (as herein defined) with at least one immunoadhesin (as defined in this application). Exemplary antibody-immunoadhesin chimeras are the bispecific CD4-IgG chimeras described in Berg *et al., PNAS (USA)* 88:4723-4727 (1991) and Chamow *et al., J. Immunol*. 153:4268 (1994).

An "isolated" molecule is one which has been identified and separated and/or recovered from a component of its natural environment. Contaminant components of its natural environment are materials which would interfere with therapeutic uses for the molecule, and may include enzymes, hormones, and other proteinaceous or nonproteinaceous solutes.

The term "cytotoxic or cytostatic agent" as used herein refers to a substance that reduces or prevents the function, or growth, of cells and/or causes destruction of cells. The term is intended to include radioactive isotopes (*e.g*., ¹³¹I ,¹²⁵I ,⁹⁰Y and ¹⁸⁶Re ), chemotherapeutic agents, and toxins such as enzymatically active toxins of bacterial, fungal, plant or animal origin, or fragments thereof.

A "chemotherapeutic agent" is a chemical compound useful in the treatment of cancer. Examples of chemotherapeutic agents include adriamycin, doxorubicin, epirubicin, 5-fluorouracil, cytosine arabinoside ("Ara-C"), cyclophosphamide, thiotepa, busulfan, cytoxin, taxoids, e.g. paclitaxel (TAXOL®, Bristol-Myers Squibb Oncology, Princeton, NJ), and docetaxel (TAXOTERE®, Rhône-Poulenc Rorer, Antony, France), methotrexate, cisplatin, melphalan, vinblastine, bleomycin, etoposide, ifosfamide, mitomycin C, mitoxantrone, vincristine, vinorelbine, carboplatin, teniposide, daunomycin, carminomycin, aminopterin, dactinomycin, mitomycins, esperamicins (see U.S. Pat. No. 4,675,187), melphalan and other related nitrogen mustards. Also included in this definition are hormonal agents that act to regulate or inhibit hormone action on tumors such as tamoxifen and onapristone.

The term "estrogen" refers to any substance (natural or synthetic) that exerts biological effects characteristic of estrogenic hormones (such as estradiol).

The term "prodrug" as used in this application refers to a precursor or derivative form of a pharmaceutically active substance that is less toxic to tumor cells compared to the parent drug and is capable of being enzymatically activated or converted into the more active parent form. See, *e.g.*, Wilman, "Prodrugs in Cancer Chemotherapy" *Biochemical Sociery Transactions,* 14, pp. 375-382, 615th Meeting Belfast (1986) and Stella *et al*., "Prodrugs: A Chemical Approach to Targeted Drug Delivery", *Directed Drug Delivery*, Borchardt *et al*., (ed.), pp. 247-267, Humana Press (1985). The prodrugs of this invention include, but are not limited to, phosphate-containing prodrugs, thiophosphate-containing prodrugs, sulfate-containing prodrugs, peptide-containing prodrugs, D-amino acid-modified prodrugs, glycosylated prodrugs, beta-lactam-containing prodrugs, optionally substituted phenoxyacetamide-containing prodrugs or optionally substituted phenylacetamide-containing prodrugs, 5-fluorocytosine and other 5-fluorouridine prodrugs which can be converted into the more active cytotoxic or cytostatic free drug. Examples of cytotoxic or cytostatic drugs that can be derivatized into a prodrug form for use in this invention include, but are not limited to, those chemotherapeutic agents described above.

A "liposome" is a small vesicle composed of various types of lipids, phospholipids and/or surfactant which is useful for delivery of a drug (such as the antagonists disclosed herein and, optionally, a chemotherapeutic agent) to a mammal. The components of the liposome are commonly arranged in a bilayer formation, similar to the lipid arrangement of biological membranes.

The terms "antisense oligodeoxynucleotide" and "antisense oligo" refer to a polynucleotide which hybridizes with an area of Eph receptor or Eph ligand mRNA or DNA and thereby prevents or reduces production of Eph receptor or Eph ligand polypeptide *in vitro* or *in vivo.* This term encompasses "modified" polynucleotides, examples of which are described herein.

### II. Modes for Carrying out the Invention

The present invention relates to the use of Eph receptor agonists and/or antagonists. Methods for making such molecules will be elaborated below. Preferably, the agonist or antagonist binds to, or interacts with, an Eph receptor (rather than Eph ligand) since, as shown in the Example below, the Eph receptor is anticipated to have a more pronounced effect on angiogensis due to potential overlap and redundancy in the Eph ligand pathway. Most preferably, the agonist or antagonist comprises, binds to, or interacts with, the EphB4 receptor and, most preferably, a human EphB4 receptor.

Where the molecule is an agonist, one may use a native sequence Eph receptor or Eph ligand (or variants or derivatives thereof) which retain one or more agonistic properties of the native receptor or ligand. Suitable EphB4 receptor molecules (HpTK5 receptor molecules) are disclosed in US patent 5,635,177 issued June 3, 1997 (expressly incorporated herein by reference) and useful Ephrin-B2 molecules (Htk ligands) are disclosed in US Patent 5,624,899, expressly incorporated herein by reference. In one embodiment, one may use a soluble Eph receptor or ligand, *e.g.* a truncated form of the receptor or ligand lacking a functional membrane anchoring region, provided the soluble molecule retains one or more agonistic properties of the native molecule. Alternatively, one may administer the gene encoding Eph receptor, Eph ligand or nucleic acid encoding agonistic variants thereof by gene therapy. Other agonist molecules are disclosed herein below.

In order to screen for agonistic molecules, one may assess the ability of a candidate molecule to stimulate tyrosine phosphorylation of the Eph receptor and/or ligand utilizing any of the various assays available for determining tyrosine phosphorylation. For example, one may expose a cell comprising the receptor or ligand (either endogenous or recombinantly introduced) to the candidate agonist and determine tyrosine phosphorylation of the receptor or ligand using an anti-phosphotyrosine antibody (see US Patent 5,635,177 issued June 3, 1997 and US Patent 5,766,863 issued June 16, 1998; the latter patent describes the "Kinase Receptor Activation Assay" which can be conveniently used to identify agonists).

Alternatively, or additionally, one may screen for other agonistic properties of a candidate agonist, *e.g.* the ability to activate downstream signaling events, *e.g.* interaction of the Eph receptor or Eph ligand with intracellular molecules.

Moreover, one may screen for the ability of a candidate agonist to stimulate angiogenesis *in vitro* or *in vivo.* For example, angiogenic activity in the avascular cornea of F344 female rat eyes may be assessed [see, *e*.*g*. Streiter *et al., Am. J. Pathol.* 141:1279 (1992)].

In an alternative embodiment, the molecule of interest is an Eph receptor antagonist. Various antagonistic molecules are described below. The preferred antagonist is a neutralizing antibody which binds to the Eph receptor and blocks binding of an Eph ligand thereto. To screen for a molecule which blocks the interaction between an Eph ligand and Eph receptor, the ligand or receptor (or a binding domain thereof) may be immobilized on a solid phase and competitive inhibition of binding of the receptor or ligand, respectively, by a candidate antagonist may be analyzed by standard techniques, such as a competitive ELISA. Alternatively, or additionally, one may screen for an antagonist which prevents or reduces tyrosine phosphorylation of the receptor or ligand using tyrosine phosphorylation assays such as those described in US Patent 5,635,177 issued June 3, 1997 or US Patent 5,766,863 issued June 16, 1998, for example. Other assays include those which assess the ability of a candidate antagonist to prevent or reduce downstream signaling events or those which prevent or reduce angiogenesis in an angiogenesis assay (see above). Alternatively, or additionally, the ability of an antagonist to eradicate or reduce tumor size in nude mice injected with tumor cells may be assessed [see, *e*.*g*., Presta *et al. Cancer Research,* 57:4593-4599 (1997)].

### A. ANTIBODIES

The present invention contemplates the use of antibodies as antagonists ("neutralizing" or "blocking" antibodies) or agonists ("agonist antibodies"). Techniques for generating antibodies will be described here. Antagonistic or agonistic properties can be screened for as described in the previous section.

### (i) Artrigen preparation

The antigen to be used for production of antibodies may be, *e*.*g*., isolated intact Eph receptor or isolated Eph ligand or one or more fragments thereof. Alternatively, cells expressing native or recombinant Eph receptor or Eph ligand may be used as immunogens for generating antibodies. The antigen may optionally further comprise a heterologous molecule, *e.g.*, an immunogenic peptide. Other forms of Eph receptor or Eph ligand useful for-generating antibodies will be apparent to those skilled in the art.

### (ii) Polyclonal antibodies

Polyclonal antibodies are preferably raised in animals by multiple subcutaneous (sc) or intraperitoneal (ip) injections of the relevant antigen and an adjuvant. It may be useful to conjugate the relevant antigen to a protein that is immunogenic in the species to be immunized, *e.g.,* keyhole limpet hemocyanin, serum albumin, bovine thyroglobulin, or soybean trypsin inhibitor using a bifunctional or derivatizing agent, for example, maleimidobenzoyl sulfosuccinimide ester (conjugation through cysteine residues), N-hydroxysuccinimide (through lysine residues), glutaraldehyde, succinic anhydride, SOCl₂, or R1N=C=NR, where R and R1 are different alkyl groups.

Animals are immunized against the antigen, immunogenic conjugates, or derivatives by combining, *e.g.*, 100 µg or 5 µg of the protein or conjugate (for rabbits or mice, respectively) with 3 volumes of Freund's complete adjuvant and injecting the solution intradermally at multiple sites. One month later the animals are boosted with 1/5 to 1/10 the original amount of peptide or conjugate in Freund's complete adjuvant by subcutaneous injection at multiple sites. Seven to 14 days later the animals are bled and the serum is assayed for antibody titer. Animals are boosted until the titer plateaus. Preferably, the animal is boosted with the conjugate of the same antigen, but conjugated to a different protein and/or through a different cross-linking reagent. Conjugates also can be made in recombinant cell culture as protein fusions. Also, aggregating agents such as alum are suitably used to enhance the immune response.

### (iii) Monoclonal antibodies

Monoclonal antibodies may be made using the hybridoma method first described by Kohler *et al., Nature,* 256:495 (1975), or may be made by recombinant DNA methods (U.S. Patent No. 4,816,567).

In the hybridoma method, a mouse or other appropriate host animal, such as a hamster or macaque monkey, is immunized as hereinabove described to elicit lymphocytes that produce or are capable of producing antibodies that will specifically bind to the protein used for immunization. Alternatively, lymphocytes may be immunized *in vitro.* Lymphocytes are then fused with myeloma cells using a suitable fusing agent, such as polyethylene glycol, to form a hybridoma cell [Goding, *Monoclonal Antibodies: Principles and Practice,* pp.59-103 (Academic Press, 1986)].

The hybridoma cells thus prepared are seeded and grown in a suitable culture medium that preferably contains one or more substances that inhibit the growth or survival of the unfused, parental myeloma cells. For example, if the parental myeloma cells lack the enzyme hypoxanthine guanine phosphoribosyl transferase (HGPRT or HPRT), the culture medium for the hybridomas typically will include hypoxanthine, aminopterin, and thymidine (HAT medium), which substances prevent the growth of HGPRT-deficient cells.

Preferred myeloma cells are those that fuse efficiently, support stable high-level production of antibody by the selected antibody-producing cells, and are sensitive to a medium such as HAT medium. Among these, preferred myeloma cell lines are murine myeloma lines, such as those derived from MOP-21 and M.C.-11 mouse tumors available from the Salk Institute Cell Distribution Center, San Diego, California USA, and SP-2 or X63-Ag8-653 cells available from the American Type Culture Collection, Rockville, Maryland USA. Human myeloma and mouse-human heteromyeloma cell lines have also been described for the production of human monoclonal antibodies [Kozbor, *J. Immunol.,* 133:3001 (1984); Brodeur *et al., Monoclonal Antibody Production Techniques and Applications,* pp. 51-63 (Marcel Dekker, Inc., New York, 1987)].

Culture medium in which hybridoma cells are growing is assayed for production of monoclonal antibodies directed against the antigen. Preferably, the binding specificity of monoclonal antibodies produced by hybridoma cells is determined by immunoprecipitation or by an *in vitro* binding assay, such as radioimmunoassay (RIA) or enzyme-linked immunoabsorbent assay (ELISA).

The binding affinity of the monoclonal antibody can, for example, be determined by the Scatchard analysis of Munson *et al., Anal. Biochem*., 107:220 (1980).

After hybridoma cells are identified that produce antibodies of the desired specificity, affinity, and/or activity, the clones may be subcloned by limiting dilution procedures and grown by standard methods [Goding, *Monoclonal Antibodies: Principles and Practice,* pp.59-103 (Academic Press, 1986)]. Suitable culture media for this purpose include, for example, D-MEM or RPMI-1640 medium. In addition, the hybridoma cells may be grown *in vivo* as ascites tumors in an animal.

The monoclonal antibodies secreted by the subclones are suitably separated from the culture medium, ascites fluid, or serum by conventional immunoglobulin purification procedures such as, for example, protein A-Sepharose, hydroxylapatite chromatography, gel electrophoresis, dialysis, or affinity chromatography.

DNA encoding the monoclonal antibodies is readily isolated and sequenced using conventional procedures (*e.g.*, by using oligonucleotide probes that are capable of binding specifically to genes encoding the heavy and light chains of the monoclonal antibodies). The hybridoma cells serve as a preferred source of such DNA. Once isolated, the DNA may be placed into expression vectors, which are then transfected into host cells such as *E. coli* cells, simian COS cells, Chinese Hamster Ovary (CHO) cells, or myeloma cells that do not otherwise produce immunoglobulin protein, to obtain the synthesis of monoclonal antibodies in the recombinant host cells.

### (iv) Humanized antibodies

A humanized antibody has one or more amino acid residues introduced into it from a source which is non-human. These non-human amino acid residues are often referred to as "import" residues, which are typically taken from an "import" variable domain. Humanization can be essentially performed following the method of Winter and co-workers [Jones *et al., Nature*, 321:522-525 (1986); Riechmann *et al., Nature,* 332:323-327 (1988); Verhoeyen *et al., Science,* 239:1534-1536 (1988)], by substituting rodent CDRs or CDR sequences for the corresponding sequences of a human antibody. Accordingly, such "humanized" antibodies are chimeric antibodies (U.S. Patent No. 4,816,567) wherein substantially less than an intact human variable domain has been substituted by the corresponding sequence from a non-human species. In practice, humanized antibodies are typically human antibodies in which some hypervariable region residues and possibly some FR residues are substituted by residues from analogous sites in rodent antibodies.

The choice of human variable domains, both light and heavy, to be used in making the humanized antibodies is very important to reduce antigenicity. According to the so-called "best-fit" method, the sequence of the variable domain of a rodent antibody is screened against the entire library of known human variable-domain sequences. The human sequence which is closest to that of the rodent is then accepted as the human FR for the humanized antibody [Sims *et al., J. Immunol.,* 151:2296 (1993)]. Another method uses a particular FR derived from the consensus sequence of all human antibodies of a particular subgroup of light or heavy chains. The same FR may be used for several different humanized antibodies [Carter *et al., Proc. Natl. Acad. Sci. USA,* 89:4285 (1992); Presta *et al., J. Immnol.,* 151:2623 (1993)].

It is further important that antibodies be humanized with retention of high affinity for the antigen and other favorable biological properties. To achieve this goal, according to a preferred method, humanized antibodies are prepared by a process of analysis of the parental sequences and various conceptual humanized products using three-dimensional models of the parental and humanized sequences. Three-dimensional immunoglobulin models are commonly available and are familiar to those skilled in the art. Computer programs are available which illustrate and display probable three-dimensional conformational structures of selected candidate immunoglobulin sequences. Inspection of these displays permits analysis of the likely role of the residues in the functioning of the candidate immunoglobulin sequence, *i.e.*, the analysis of residues that influence the ability of the candidate immunoglobulin to bind its antigen. In this way, FR residues can be selected and combined from the recipient and import sequences so that the desired antibody characteristic, such as increased affinity for the target antigen(s), is achieved. In general, the hypervariable region residues are directly and most substantially involved in influencing antigen binding.

### (v) Human antibodies

As an alternative to humanization, human antibodies can be generated. For example, it is now possible to produce transgenic animals (*e.g.*, mice) that are capable, upon immunization, of producing a full repertoire of human antibodies in the absence of endogenous immunoglobulin production. For example, it has been described that the homozygous deletion of the antibody heavy-chain joining region (JH) gene in chimeric and germ-line mutant mice results in complete inhibition of endogenous antibody production. Transfer of the human germ-line immunoglobulin gene array in such germ-line mutant mice will result in the production of human antibodies upon antigen challenge. See, *e.g.*, Jakobovits *et al., Proc. Natl. Acad. Sci. USA,* 90:2551 (1993); Jakobovits *et al., Nature,* 362:255-258 (1993); Bruggermann *et al., Year in Immuno*., 7:33 (1993); and US Patents 5,591,669, 5,589,369 and 5,545,807. Human antibodies can also be derived from phage-display libraries [Hoogenboom *et al., J. Mol. Biol.,* 227:381 (1991); Marks *et al., J. Mol. Biol.,* 222:581-597 (1991); and US Patents 5,565,332 and 5,573,905]. Human antibodies may also be generated by *in vitro* activated B cells (see, *e.g.,* US Patents 5,567,610 and 5,229,275).

### (vi) Antibody fragments

Various techniques have been developed for the production of antibody fragments. Traditionally, these fragments were derived via proteolytic digestion of intact antibodies [see, *e.g.,* Morimoto *et al., Journal of Biochemical and Biophysical Methods* 24:107-117 (1992) and Brennan *et al., Science* 229:81 (1985)]. However, these fragments can now be produced directly by recombinant host cells. For example, Fab'-SH fragments can be directly recovered from E. *coli* and chemically coupled to form F(ab')₂ fragments [Carter *et al., Bio*/*Technology* 10:163-167 (1992)]. In another embodiment, the F(ab')₂ is formed using the leucine zipper GCN4 to promote assembly of the F(ab')₂ molecule. According to another approach, Fv, Fab or F(ab')₂ fragments can be isolated directly from recombinant host cell culture. Other techniques for the production of antibody fragments will be apparent to the skilled practitioner.

### (vii) Multispecific antibodies

*In* some embodiments, it may be desirable to generate multispecific *(e.g.* bispecific) antibodies having binding specificities for at least two different epitopes. Exemplary bispecific antibodies may bind to two different epitopes on the target antigen. Alternatively, an anti-Eph ligand or anti-Eph receptor arm may be combined with an arm which binds to a triggering molecule on a leukocyte such as a T-cell receptor molecule (*e.g.,* CD2 or CD3), or Fc receptors for IgG (FcγR), such as FcγRI (CD64), FcγRII (CD32) and FcγRIII (CD16) so as to focus cellular defense mechanisms to the Eph receptor or Eph ligand-expressing cell. Bispecific antibodies may also be used to localize cytotoxic agents to cells which express Eph receptor or Eph ligand. These antibodies possess an Eph receptor or Eph ligand-binding arm and an arm which binds the cytotoxic agent (e.g., saporin, vinca alkaloid, ricin A chain, methotrexate or radioactive isotope hapten).

In another embodiment, the bispecific antibody has one arm which binds an Eph ligand and another arm which binds an Eph receptor (for example, the cognate receptor to which the endogenous ligand binds). Bispecific antibodies which are capable of cross-linking different Eph ligands or different Eph receptors are also contemplated. Such bispecific antibodies may be employed as agonists or antagonists, depending on their biological activities.

Bispecific antibodies can be prepared as full length antibodies or antibody fragments (*e.g.*, F(ab')₂ bispecific antibodies).

According to one approach for making preferably full length bispecific antibodies, the interface between a pair of antibody molecules can be engineered to maximize the percentage of heterodimers which are recovered from recombinant cell culture. The preferred interface comprises at least a part of the CH3 domain of an antibody constant domain. In this method, one or more small amino acid side chains from the interface of the first antibody molecule are replaced with larger side chains (*e.g.*, tyrosine or tryptophan). Compensatory "cavities" of identical or similar size to the large side chain(s) are created on the interface of the second antibody molecule by replacing large amino acid side chains with smaller ones *(e.g.,* alanine or threonine). This provides a mechanism for increasing the yield of the heterodimer over other unwanted end-products such as homodimers. See US Patent 5,731,168 issued March 24, 1998.

Bispecific antibodies include cross-linked or "heteroconjugate" antibodies. For example, one of the antibodies in the heteroconjugate can be coupled to avidin, the other to biotin. Heteroconjugate antibodies may be made using any convenient cross-linking methods. Suitable cross-linking agents are well known in the art, and are disclosed in US Patent No. 4,676,980, along with a number of cross-linking techniques.

Techniques for generating bispecific antibodies from antibody fragments have also been described in the literature. For example, bispecific antibodies can be prepared using chemical linkage. See, for example, Brennan *et al., Science* 229:81 (1985) and Shalaby *et al., J. Exp. Med.* 175:217-225 (1992).

Various techniques for making and isolating bispecific antibody fragments directly from recombinant cell culture have also been described. For example, bispecific antibodies have been produced using leucine zippers. Kosielny *et al., J. Immunol*. 148(5):1547-1553 (1992). The leucine zipper peptides from the Fos and Jun proteins were linked to the Fab' portions of two different antibodies by gene fusion. The antibody homodimers were reduced at the hinge region to form monomers and then re-oxidized to form the antibody heterodimers. This method can also be utilized for the production of antibody homodimers. The "diabody" technology described by *Hollinger et al., Proc. Natl. Acad. Sci. USA* 90:6444-6448 (1993) has provided an alternative mechanism for making bispecific antibody fragments. The fragments comprise a heavy-chain variable domain (VH) connected to a light-chain variable domain (VL) by a linker which is too short to allow pairing between the two domains on the same chain. Accordingly, the VH and VL domains of one fragment are forced to pair with the complementary VL and VH domains of another fragment, thereby forming two antigen-binding sites. Another strategy for making bispecific antibody fragments by the use of single-chain Fv (sFv) dimers has also been reported. See Gruber *et al., J. Immunol*. 152:5368 (1994). Alternatively, the bispecific antibody may be a "linear antibody" produced as described in US Patent 5,641,870 issued June 24, 1997, for example.

Antibodies with more than two valencies are contemplated. For example, trispecific antibodies can be prepared. Tutt *et al., J. Immunol*. 147:60 (1991).

### (viii) Other modifications

Other modifications of the antibody are contemplated. For example, it may be desirable to modify the antibody of the invention with respect to effector function, so as to enhance the effectiveness of the antibody in treating cancer, for example. For example cysteine residue(s) may be introduced in the Fc region, thereby allowing interchain disulfide bond formation in this region. The homodimeric antibody thus generated may have improved internalization capability and/or increased complement-mediated cell killing and antibody-dependent cellular cytotoxicity (ADCC). See *Caron et al., J. Exp Med*. 176:1191-1195 (1992) and Shopes, B. *J. Immunol*. 148:2918-2922 (1992). Homodimeric antibodies with enhanced anti-tumor activity may also be prepared using heterobifunctional cross-linkers as described in Wolff *et al., Cancer Research* 53:2560-2565 (1993). Alternatively, an antibody can be engineered which has dual Fc regions and may thereby have enhanced complement lysis and ADCC capabilities. See Stevenson *et al., Anti-Cancer Drug Design* 3:219-230 (1989).

In certain embodiments of the invention, it may be desirable to use an antibody fragment, rather than an intact antibody, to increase tumor penetration, for example. In this case, it may be desirable to modify the antibody fragment in order to increase its serum half life. This may be achieved, for example, by incorporation of a salvage receptor binding epitope into the antibody fragment (*e.g.*, by mutation of the appropriate region in the antibody fragment or by incorporating the epitope into a peptide tag that is then fused to the antibody fragment at either end or in the middle, *e.g.,* by DNA or peptide synthesis). See US Patent 5,739,277.

### B. POLYPEPTIDE DERIVATIVES OR VARIANTS

Other useful antagonists or agonists include amino acid sequence variants and/or derivatives of native Eph receptor or native Eph ligand that bind to an endogenous receptor or ligand and competitively inhibit interaction between the native ligand and receptor (antagonists) or activate the receptor or ligand (agonists).

For example, such antagonists include fragments and amino acid sequence variants that comprise a receptor binding domain of an Eph ligand or a ligand binding domain of an Eph receptor, but that lack a domain conferring biological activity, or that otherwise are defective in activating cellular Eph receptors or ligands.

Receptor binding domains in Eph ligand and ligand binding domains in Eph receptor are determined by methods known in the art, including X-ray studies, mutational analyses, and antibody binding studies. The mutational approaches include the techniques of random saturation mutagenesis coupled with selection of escape mutants, and insertional mutagenesis. Another strategy suitable for identifying binding domains is known as alanine (Ala)-scanning mutagenesis. Cunningham, *et al., Science* 244:1081-1985 (1989). This method involves the identification of regions that contain charged amino acid side chains. The charged residues in each region identified (*i*.*e*. Arg, Asp, His, Lys, and Glu) are replaced (one region per mutant molecule) with Ala and the binding of the obtained variants is tested, to assess the importance of the particular region in binding. A further powerful method for the localization of binding domains is through the use of neutralizing antibodies. Usually a combination of these and similar methods is used for localizing the domains involved in receptor or ligand binding.

Substitutional variants are those that have at least one amino acid residue in a native sequence removed and a different amino acid inserted in its place at the same position. The substitutions may be single, where only one amino acid in the molecule has been substituted, or they may be multiple, where two or more amino acids have been substituted in the same molecule.

Insertional variants are those with one or more amino acids inserted immediately adjacent to an amino acid at a particular position in a native sequence. Immediately adjacent to an amino acid means connected to either the α-carboxy or α-amino functional group of the amino acid.

Deletional variants are those with one or more amino acid residues in a native sequence removed. Ordinarily, deletional variants will have one or two amino acid residues deleted in a particular region of the molecule. A preferred deletional variant is a soluble Eph receptor or Eph ligand, optionally fused to a heterologous molecule, such as an immunoglobulin region (see section on "Immunoadhesins" below) or other molecule, *e.g.* polyethylene glycol.

Fragments and amino acid sequence variants are readily prepared by methods known in the art, such as by site directed mutagenesis of the DNA encoding the native factor. The mutated DNA is inserted into an appropriate expression vector, and host cells are then transfected with the recombinant vector. The recombinant host cells are grown in suitable culture medium, and the desired fragment or amino acid sequence variant expressed in the host cells is then recovered from the recombinant cell culture by chromatographic or other purification methods.

Alternatively, fragments and amino acid variants are prepared *in vitro*, for example by proteolysis of native Eph receptor or Eph ligand, or by synthesis using standard solid-phase peptide synthesis procedures as described by Merrifield *J. Am. Chem. Soc.* 85:2149 (1963), although other equivalent chemical syntheses known in the art may be used. Solid-phase synthesis is initiated from the C-terminus of the peptide by coupling a protected α-amino acid to a suitable resin. The amino acids are coupled to the peptide chain using techniques well known in the art for the formation of peptide bonds.

### C. IMMUNOADHESINS

One preferred derivative of the Eph receptor or Eph ligand is an immunoadhesin. The immunoadhesin may be an agonist or antagonist. The immunoadhesin antagonist may comprise a portion of the Eph receptor which binds an Eph ligand (or vice versa) and therefore serves to competitively inhibit the interaction between endogenous receptor and ligand. Where oligomerization or transphosphorylation is involved in receptor or ligand activation, for example, an immunoadhesin comprising two or more ligand or receptor binding domains in the appropriate configuration may act as a receptor or ligand agonist.

The simplest and most straightforward immunoadhesin design combines the binding domain(s) of the adhesin (*e.g.* the extracellular domain (ECD) of an Eph receptor or Eph ligand) with the hinge and Fc regions of an immunoglobulin heavy chain. Ordinarily, when preparing the immunoadhesins of the present invention, nucleic acid encoding the binding domain of the adhesin will be fused C-terminally to nucleic acid encoding the N-terminus of an immunoglobulin constant domain sequence, however N-terminal fusions are also possible.

Typically, in such fusions the encoded chimeric polypeptide will retain at least functionally active hinge, CH2 and CH3 domains of the constant region of an immunoglobulin heavy chain. Fusions are also made to the C-terminus of the Fc portion of a constant domain, or immediately N-terminal to the CH1 of the heavy chain or the corresponding region of the light chain. The precise site at which the fusion is made is not critical; particular sites are well known and may be selected in order to optimize the biological activity, secretion, or binding characteristics of the immunoadhesin.

In a preferred embodiment, the adhesin sequence is fused to the N-terminus of the Fc domain of immunoglobulin G1 (IgG1). It is possible to fuse the entire heavy chain constant region to the adhesin sequence. However, more preferably, a sequence beginning in the hinge region just upstream of the papain cleavage site which defines IgG Fc chemically (*i*.*e*. residue 216, taking the first residue of heavy chain constant region to be 114), or analogous sites of other immunoglobulins is used in the fusion. In a particularly preferred embodiment, the adhesin amino acid sequence is fused to (a) the hinge region and CH2 and CH3 or (b) the CH1, hinge, CH2 and CH3 domains, of an IgG heavy chain.

For bispecific immunoadhesins, the immunoadhesins are assembled as multimers, and particularly as heterodimers or heterotetramers. Generally, these assembled immunoglobulins will have known unit structures. A basic four chain structural unit is the form in which IgG, IgD, and IgE exist. A four chain unit is repeated in the higher molecular weight immunoglobulins; IgM generally exists as a pentamer of four basic units held together by disulfide bonds. IgA globulin, and occasionally IgG globulin, may also exist in multimeric form in serum. In the case of multimers, each of the four units may be the same or different.

Various exemplary assembled immunoadhesins within the scope herein are schematically diagrammed below:
(a) AC_{L}-AC_{L};
(b) AC_{H}-(AC_{H}, AC_{L} - AC_{H}, AC_{L} -V_{H}C_{H}, or V_{L}C_{L}- AC_{H});
(c) AC_{L} - AC_{H} -(AC_{L} - AC_{H}, AC_{L} - V_{H}C_{H}, V_{L}C_{L}- AC_{H}, or V_{L}C_{L}- V_{H}C_{H})
(d) AC_{L} - V_{H}C_{H} -( AC_{H}, or AC_{L} - V_{H}C_{H}, or V_{L}C_{L}- AC_{H});
(e) V_{L}C_{L}- AC_{H} -(AC_{L} - V_{H}C_{H}, or V_{L}C_{L}-AC_{H}); and
(f) (A-Y)ₙ-(V_{L}C_{L}- V_{H}C_{H})₂,
wherein each A represents identical or different adhesin amino acid sequences;
V_{L} is an immunoglobulin light chain variable domain;
V_{H} is an immunoglobulin heavy chain variable domain;
C_{L} is an immunoglobulin light chain constant domain;
C_{H} is an immunoglobulin heavy chain constant domain;
n is an integer greater than 1;
Y designates the residue of a covalent cross-linking agent.

In the interests of brevity, the foregoing structures only show key features; they do not indicate joining (J) or other domains of the immunoglobulins, nor are disulfide bonds shown. However, where such domains are required for binding activity, they shall be constructed to be present in the ordinary locations which they occupy in the immunoglobulin molecules.

Alternatively, the adhesin sequences can be inserted between immunoglobulin heavy chain and light chain sequences, such that an immunoglobulin comprising a chimeric heavy chain is obtained. In this embodiment, the adhesin sequences are fused to the 3' end of an immunoglobulin heavy chain in each arm of an immunoglobulin, either between the hinge and the C_{H}2 domain, or between the C_{H}2 and C_{H}3 domains. Similar constructs have been reported by Hoogenboom, *et al., Mol. Immunol*. 28:1027-1037 (1991).

Although the presence of an immunoglobulin light chain is not required in the immunoadhesins of the present invention, an immunoglobulin light chain might be present either covalently associated to an adhesin-immunoglobulin heavy chain fusion polypeptide, or directly fused to the adhesin. In the former case, DNA encoding an immunoglobulin light chain is typically coexpressed with the DNA encoding the adhesin-immunoglobulin heavy chain fusion protein. Upon secretion, the hybrid heavy chain and the light chain will be covalently associated to provide an immunoglobulin-like structure comprising two disulfide-linked immunoglobulin heavy chain-light chain pairs. Methods suitable for the preparation of such structures are, for example, disclosed in U.S. Patent No. 4,816,567, issued 28 March 1989.

Immunoadhesins are most conveniently constructed by fusing the cDNA sequence encoding the adhesin portion in-frame to an immunoglobulin cDNA sequence. However, fusion to genomic immunoglobulin fragments can also be used [see, *e.g.* Aruffo *et al., Cell* 61:1303-1313 (1990); and Stamenkovic *et al.,* Cell 66:1133-1144 (1991)]. The latter type of fusion requires the presence of Ig regulatory sequences for expression. cDNAs encoding IgG heavy-chain constant regions can be isolated based on published sequences from cDNA libraries derived from spleen or peripheral blood lymphocytes, by hybridization or by polymerase chain reaction (PCR) techniques. The cDNAs encoding the "adhesin" and the immunoglobulin parts of the immunoadhesin are inserted in tandem into a plasmid vector that directs efficient expression in the chosen host cells.

### D. OTHER AGONIST OR ANTAGONIST MOLECULES

The present invention contemplates additional antagonists or agonists.

For example, the antagonist or agonist may be a synthetic small molecule. For example, the small molecule may mimic the binding site of the Eph receptor or Eph ligand and thereby act as an agonist or antagonist. Alternatively, the small molecule may interact with the tyrosine kinase domain of the receptor or ligand and thereby activate or prevent activation of the tyrosine kinase domain. See, *e.g.*, US Patent 5,795,910.

The antagonist or agonist may also be a peptide generated by rational design or by phage display (WO98/35036 published 13 August 1998), for example. Thus, the molecule of choice may be a "CDR mimic" or antibody analogue designed based on the CDRs of an antibody, for example.

Alternatively, the agonist or antagonist may interact with the promoter or other regulatory region of an *Eph* gene, essentially following the approach in WO95/28485 published 26 October 1995, for example.

Another agonist of interest is a molecule such as a dimer comprising two or more binding sites of an Eph receptor or Eph ligand. Such molecules may be formed by fusing the binding domain to a dimerization region which promotes stable interaction of the domains in the dimer. Such molecules may be useful agonists for promoting "receptor cross-talk" or transphosphorylation of Eph receptors or Eph ligands.

Another type of antagonist is an antisense nucleic acid. Antisense inhibition of gene expression can occur at multiple levels. The preferred approach however, is one which involves interfering with translation of mRNA into protein. To achieve this, one may use an oligodeoxynucleotide (oligo) complementary to the Eph receptor or Eph ligand mRNA. This antisense oligo binds by complementary Watson-Crick base pairing to the native sense mRNA. However, plasmid-derived antisense RNA (*i*.*e*. wherein the antisense DNA is provided in a plasmid) is also contemplated. Mercola and Cohen *Cancer Gene Therapy* 2(1):47-59 (1995). According to another embodiment, the so-called "anti-gene approach", the antisense molecule is one which binds to form a triple helix or triplex with Eph receptor or Eph ligand DNA via Hoogsteen (or anti-Hoogsteen) hydrogen bonding of the third base in the antisense molecule with the already formed pair. Mercola and Cohen, *supra*. While antisense oligos can be directed anywhere along the mRNA transcript, the preferred target sequence is at the 5' end thereof, spanning the initiation codon. Oligos of interest will normally comprise at least 15-17 bases. To identify the most active antisense sequence, deletion analysis may be performed.

Antisense oligos can be readily synthesized by automated methods. See US Patent No. 5,489,677 issued Feb 6, 1996. Normally the antisense oligo intended for *in vivo* use will be modified so as to render it less susceptible to nuclease degradation and/or to improve the efficiency with which it is taken up by a cell. Several backbone modifications have been developed to counteract nuclease degradation. One exemplary modification results in a "methyl-phosphonate" (MO) oligo. According to this approach, one of the nonbridging oxygen atoms in the internucleotide bond is replaced with a methyl group which has the net effect of eliminating the negative charge of the oligo. Tonkinson *et al. Cancer Investigation* 14(1):54-65 (1996). Another modification described in Tonkinson *et al*. for reducing nuclease degradation is the phosphorothioate (PS) modification which involves replacing one of the nonbridging oxygens at the phosphorus with a sulfur. Other ways of modifying the basic oligo are reviewed in Cohen, J. *Adv. Pharmacol*. 25:319-339 (1993). For example, a new analog has been reported wherein the entire deoxyribose-phosphate backbone was replaced with a peptide-like backbone. See Mercola and Cohen, *supra*. To improve cellular uptake, the antisense oligos can be encapsulated in liposomes, complexed with cationic lipids such as DOTMA, coupled to polylysine or lipofectin, and/or covalently attached to a cholesteryl moiety. See Tonkinson *et al., supra*.

Aside from antisense nucleic acids, as noted above, the present invention also contemplates administration of nucleic acid encoding any agonist or antagonist as herein-described. For example, the present invention contemplates "intracellular antibodies" introduced into the cell by gene therapy.

There are two major approaches to getting the nucleic acid (optionally contained in a vector) into the patient's cells; *in vivo* and *ex vivo.* For *in vivo* delivery the nucleic acid is injected directly into the patient, usually at the site where the agonist or antagonist is required. For *ex vivo* treatment, the patient's cells are removed, the nucleic acid is introduced into these isolated cells and the modified cells are administered to the patient either directly or, for example, encapsulated within porous membranes which are implanted into the patient (see, *e.g.* U.S. Patent Nos. 4,892,538 and 5,283,187). There are a variety of techniques available for introducing nucleic acids into viable cells. The techniques vary depending upon whether the nucleic acid is transferred into-cultured cells *in vitro*, or *in vivo* in the cells of the intended host. Techniques suitable for the transfer of nucleic acid into mammalian cells *in vitro* include the use of liposomes, electroporation, microinjection, cell fusion, DEAE-dextran, the calcium phosphate precipitation method, etc. A commonly used vector for *ex vivo* delivery of the gene is a retrovirus.

The currently preferred *in vivo* nucleic acid transfer techniques include transfection with viral vectors (such as adenovirus, Herpes simplex I virus, or adeno-associated virus) and lipid-based systems (useful lipids for lipid-mediated transfer of the gene are DOTMA, DOPE and DC-Chol, for example). In some situations it is desirable to provide the nucleic acid source with an agent that targets the target cells, such as an antibody specific for a cell surface membrane protein or the target cell, a ligand for a receptor on the target cell, etc. Where liposomes are employed, proteins which bind to a cell surface membrane protein associated with endocytosis may be used for targeting and/or to facilitate uptake, *e.g.* capsid proteins or fragments thereof tropic for a particular cell type, antibodies for proteins which undergo internalization in cycling, and proteins that target intracellular localization and enhance intracellular half-life. The technique of receptor-mediated endocytosis is described, for example, by Wu *et al., J. Biol. Chem*. 262:4429-4432 (1987); and Wagner *et al., Proc. Natl. Acad. Sci. USA* 87:3410-3414 (1990). For review of the currently known gene marking and gene therapy protocols see Anderson *et al., Science* 256:808-813 (1992). See also WO 93/25673and the references cited therein.

### E. CONJUGATED MOLECULES

Methods utilizing "conjugates" of the antagonists or agonists described above and heterologous molecules are also contemplated. The antagonist or agonist and heterologous molecule(s) are linked in a variety of different ways including chemical cross-linking, production of fusion proteins, etc.

A preferred conjugate herein comprises an agonist or antagonist conjugated to a molecule which increases its serum half-life. For example, one may link the agonist or antagonist to any one of a variety of nonproteinaceous polymers, *e*.*g*., polyethylene glycol, polypropylene glycol, or polyoxyalkylenes, in the manner set forth in U.S. Patent Nos. 4,640,835; 4,496,689; 4,301,144; 4,670,417; 4,791,192 or 4,179,337.

Another preferred conjugate comprises an antagonist conjugated to a cytotoxic or cytostatic agent. Exemplary cytotoxic or cytostatic agents useful for generating such constructs include, without limitation, chemotherapeutic agents, toxins (*e*.*g*., an enzymatically active toxin of bacterial, fungal, plant or animal origin, or fragments thereof), or radioactive isotopes (*i*.*e*., a radioconjugate).

Chemotherapeutic agents useful in the generation of such conjugates have been described above. Enzymatically active toxins and fragments thereof which can be used include diphtheria A chain, nonbinding active fragments of diphtheria toxin, exotoxin A chain (from *Pseudomonas aeruginosa*), ricin A chain, abrin A chain, modeccin A chain, alpha-sarcin, *Aleurites fordii* proteins, dianthin proteins, *Phytolaca americana* proteins (PAPI, PAPII, and PAP-S), *momordica charantia* inhibitor, curcin, crotin, *sapaonaria officinalis* inhibitor, gelonin, mitogellin, restrictocin, phenomycin, enomycin and the tricothecenes. A variety of radionuclides are available for the production of radioconjugated antagonists. Examples include ²¹²Bi, ¹³¹I, ¹³¹In, ⁹⁰Y and ¹⁸⁶Re.

Conjugates of the antagonist and cytotoxic or cytostatic agent are made using a variety of bifunctional protein coupling agents such as N-succinimidyl-3-(2-pyridyldithiol) propionate (SPDP), iminothiolane (IT), bifunctional derivatives of imidoesters (such as dimethyl adipimidate HCI), active esters (such as disuccinimidyl suberate), aldehydes (such as glutareldehyde), bis-azido compounds (such as bis (p-azidobenzoyl) hexanediamine), bis-diazonium derivatives (such as bis-(p-diazoniumbenzoyl)-ethylenediamine), diisocyanates (such as toluene 2,6-diisocyanate), and bis-active fluorine compounds (such as 1,5-difluoro-2,4-dinitrobenzene). For example, a ricin immunotoxin can be prepared as described in Vitetta *et al., Science* 238:1098 (1987). Carbon-14-labeled 1-isothiocyanatobenzyl-3-methyldiethylene triaminepentaacetic acid (MX-DTPA) is an exemplary chelating agent for conjugation of radionucleotide to the antibody. See WO94/11026. Alternatively, fusion proteins comprising a polypeptide antagonist and cytotoxic or cytostatic polypeptide can be prepared. Such molecules can also be generated by peptide synthesis, for example.

In another embodiment, the antagonist may be conjugated to a "receptor" (such as streptavidin) for utilization in tumor pre-targeting wherein the antagonist-receptor conjugate is administered to the patient, followed by removal of unbound conjugate from the circulation using a clearing agent and then administration of a "ligand" *(e.g.,* avidin) which is conjugated to a cytotoxic or cytostatic agent *(e.g.,* a radionuclide).

The antagonists of the present invention may also be used in ADEPT by conjugating the antagonist to a prodrug-activating enzyme which converts a prodrug (*e.g.,* a peptidyl chemotherapeutic agent, see WO81/01145) to an active anti-cancer drug. See, for example, WO 88/07378 and U.S. Patent No. 4,975,278.

The enzyme component of the antagonist conjugate useful for ADEPT includes any enzyme capable of acting on a prodrug in such a way so as to convert it into its more active, cytotoxic form.

Enzymes that are useful in the method of this invention include, but are not limited to, alkaline phosphatase useful for converting phosphate-containing prodrugs into free drugs; arylsulfatase useful for converting sulfate-containing prodrugs into free drugs; cytosine deaminase useful for converting non-toxic 5-fluorocytosine into the anti-cancer drug, 5-fluorouracil; proteases, such as serratia protease, thermolysin, subtilisin, carboxypeptidases and cathepsins (such as cathepsins B and L), that are useful for converting peptide-containing prodrugs into free drugs; D-alanylcarboxypeptidases, useful for converting prodrugs that contain D-amino acid substituents; carbohydrate-cleaving enzymes such as beta-galactosidase and neuraminidase useful for converting glycosylated prodrugs into free drugs; beta-lactamase useful for converting drugs derivatized with β-lactams into free drugs; and penicillin, such as penicillin V amidase or penicillin G amidase, useful for converting drugs derivatized at their amine nitrogens with phenoxyacetyl or phenylacetyl groups, respectively, into free drugs. Alternatively, antibodies with enzymatic activity, also known in the art as "abzymes", can be used to convert the prodrugs of the invention into free active drugs [see, *e.g.,* Massey, *Nature* 328:457-458 (1987)]. Antibody-abzyme conjugates can be prepared as described herein for delivery of the abzyme to a tumor cell population.

The enzymes of this invention can be covalently bound to the antagonist by techniques well known in the art such as the use of the heterobifunctional crosslinking reagents discussed above. Alternatively, fusion proteins comprising at least the Eph receptor or Eph ligand-binding domain of an antagonist of the invention linked to at least a functionally active portion of an enzyme of the invention can be constructed using recombinant DNA techniques well known in the art [see, *e.g.,* Neuberger *et al., Nature* 312:604-608 (1984)].

### F. PHARMACEUTICAL COMPOSITIONS

Therapeutic formulations of the agonist or antagonist are prepared for storage by mixing the agonist or antagonist having the desired degree of purity with optional physiologically acceptable carriers, excipients or stabilizers [*Remington's Pharmaceutical Sciences* 16th edition, Osol, A. Ed. (1980)], in the form of lyophilized formulations or aqueous solutions. Acceptable carriers, excipients, or stabilizers are nontoxic to recipients at the dosages and concentrations employed, and include buffers such as phosphate, citrate, and other organic acids; antioxidants including ascorbic acid and methionine; preservatives (such as octadecyldimethylbenzyl ammonium chloride; hexamethonium chloride; benzalkonium chloride, benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counterions such as sodium; metal complexes (*e*.*g*., Zn-protein complexes); and/or non-ionic surfactants such as TWEEN™, PLURONICS™ or polyethylene glycol (PEG).

The agonists or antagonists may also be formulated in liposomes. Liposomes containing the molecule of interest are prepared by methods known in the art, such as described in Epstein *et al., Proc. Natl. Acad. Sci*. USA 82:3688 (1985); Hwang *et al., Proc. Natl Acad. Sci. USA* 77:4030 (1980); and U.S. Pat. Nos. 4,485,045 and 4,544,545. Liposomes with enhanced circulation time are disclosed in U.S. Patent No. 5,013,556.

Particularly useful immunoliposomes can be generated by the reverse phase evaporation method with a lipid composition comprising phosphatidylcholine, cholesterol and PEG-derivatized phosphatidylethanolamine (PEG-PE). Liposomes are extruded through filters of defined pore size to yield liposomes with the desired diameter. Fab' fragments of an antibody of the present invention can be conjugated to the liposomes as described in Martin *et al., J. Biol. Chem*. 257:286-288 (1982) via a disulfide interchange reaction. A chemotherapeutic agent (such as Doxorubicin) is optionally contained within the liposome. See Gabizon *et al., J. National Cancer Inst*.81(19):1484 (1989)

The formulation herein may also contain more than one active compound as necessary for the particular indication being treated, preferably those with complementary activities that do not adversely affect each other. Such molecules are suitably present in combination in amounts that are effective for the purpose intended. For example, an Eph receptor antagonist may be combined with a chemotherapeutic agent or estrogen.

The active ingredients may also be entrapped in microcapsules prepared, for example, by coacervation techniques or by interfacial polymerization, for example, hydroxymethylcellulose or gelatin-microcapsule and poly-(methylmethacylate) microcapsules, respectively, in colloidal drug delivery systems (for example, liposomes, albumin microspheres, microemulsions, nano-particles and nanocapsules) or in macroemulsions. Such techniques are disclosed in *Remington's Pharmaceutical Sciences* 16th edition, Osol, A. Ed. ( 1980).

The formulations to be used for in vivo administration must be sterile. This is readily accomplished by filtration through sterile filtration membranes.

Sustained-release preparations may be prepared. Suitable examples of sustained-release preparations include semipermeable matrices of solid hydrophobic polymers containing the antagonist, which matrices are in the form of shaped articles, *e*.*g*., films, or microcapsules. Examples of sustained-release matrices include polyesters, hydrogels [for example, poly(2-hydroxyethyl-methacrylate), or poly(vinylalcohol)], polylactides (U.S. Pat. No. 3,773,919), copolymers of L-glutamic acid and ethyl-L-glutamate, non-degradable ethylene-vinyl acetate, degradable lactic acid-glycolic acid copolymers such as the Lupron Depot™ (injectable microspheres composed of lactic acid-glycolic acid copolymer and leuprolide acetate), and poly-D-(-)-3-hydroxybutyric acid. While polymers such as ethylene-vinyl acetate and lactic acid-glycolic acid enable release of molecules for over 100 days, certain hydrogels release proteins for shorter time periods. When encapsulated antibodies remain in the body for a long time, they may denature or aggregate as a result of exposure to moisture at 37°C, resulting in a loss of biological activity and possible changes in immunogenicity. Rational strategies can be devised for stabilization depending on the mechanism involved. For example, if the aggregation mechanism is discovered to be intermolecular S-S bond formation through thio-disulfide interchange, stabilization may be achieved by modifying sulfhydryl residues, lyophilizing from acidic solutions, controlling moisture content, using appropriate additives, and developing specific polymer matrix compositions.

### G. THERAPY WITH EPH RECEPTOR ANTAGONISTS

For therapeutic applications, the antagonists of the invention are administered to a mammal, preferably a human, in a pharmaceutically acceptable dosage form such as those discussed above, including those that may be administered to a human intravenously as a bolus or by continuous infusion over a period of time, by intramuscular, intraperitoneal, intracerebrospinal, subcutaneous, intraarticular, intrasynovial, intrathecal, oral, topical, or inhalation routes. The antagonists are also suitably administered by intratumoral, peritumoral, intralesional, or perilesional routes, to exert local as well as systemic therapeutic effects. The intraperitoneal route is expected to be particularly useful, for example, in the treatment of ovarian tumors.

For the prevention or treatment of disease, the appropriate dosage of antagonist will depend on the type of disease to be treated, as defined above, the severity and course of the disease, whether the antagonist is administered for preventive or therapeutic purposes, previous therapy, the patient's clinical history and response to the antagonist, and the discretion of the attending physician. The antagonist is suitably administered to the patient at one time or over a series of treatments.

The antagonists are useful in the treatment of various neoplastic and non-neoplastic diseases and disorders. Cancers and related conditions that are amenable to treatment include breast carcinomas, lung carcinomas, gastric carcinomas, esophageal carcinomas, colorectal carcinomas, liver carcinomas, ovarian carcinomas, thecomas, arrhenoblastomas, cervical carcinomas, endometrial carcinoma, endometrial hyperplasia, endometriosis, fibrosarcomas, choriocarcinoma, head and neck cancer, nasopharyngeal carcinoma, laryngeal carcinomas, hepatoblastoma, Kaposi's sarcoma, melanoma, skin carcinomas, hemangioma, cavernous hemangioma, hemangioblastoma, pancreas carcinomas, retinoblastoma, astrocytoma, glioblestoma, Schwannoma, oligodendroglioma, medulloblastoma, neuroblastomas, rhabdomyosarcoma, osteogenic sarcoma, leiomyosarcomas, urinary tract carcinomas, thyroid carcinomas, Wilm's tumor, renal cell carcinoma, prostate carcinoma, abnormal vascular proliferation associated with phakomatoses, edema (such as that associated with brain tumors), and Meigs' syndrome.

Non-neoplastic conditions that are amenable to treatment include rheumatoid arthritis, psoriasis, atherosclerosis, diabetic and other proliferative retinopathies including retinopathy of prematurity, retrolental fibroplasia, neovascular glaucoma, age-related macular degeneration, thyroid hyperplasias (including Grave's disease), corneal and other tissue transplantation, chronic inflammation, lung inflammation, nephrotic syndrome, preeclampsia, ascites, pericardial effusion (such as that associated with pericarditis), and pleural effusion.

Depending on the type and severity of the disease, about 1 µg/kg to about 50 mg/kg (*e.g.*, 0. 1-20mg/kg) of antagonist is an initial candidate dosage for administration to the patient, whether, for example, by one or more separate administrations, or by continuous infusion. A typical daily or weekly dosage might range from about 1 µg/kg to about 20 mg/kg or more, depending on the factors mentioned above. For repeated administrations over several days or longer, depending on the condition, the treatment is repeated until a desired suppression of disease symptoms occurs. However, other dosage regimens may be useful. The progress of this therapy is easily monitored by conventional techniques and assays, including, for example, radiographic tumor imaging.

According to another embodiment of the invention, the effectiveness of the antagonist in preventing or treating disease may be improved by administering the antagonist serially or in combination with another agent that is effective for those purposes, such as tumor necrosis factor (TNF), an antagonist capable of inhibiting or neutralizing the angiogenic activity of acidic or basic fibroblast growth factor (FGF) or hepatocyte growth factor (HGF), an antagonist capable of inhibiting or neutralizing the coagulant activities of tissue factor, protein C, or protein S (see Esmon *et al*., PCT Patent Publication No. WO 91/01753, published 21 February 1991), an antagonist such as an antibody capable of binding to HER2 receptor (see US Patent 5,772,997), or one or more conventional therapeutic agents such as, for example, alkylating agents, folic acid antagonists, anti-metabolites of nucleic acid metabolism, antibiotics, pyrimidine analogs, 5-fluorouracil, cisplatin, purine nucleosides, amines, amino acids, triazole nucleosides, or corticosteroids. Such other agents may be present in the composition being administered or may be administered separately. Also, the antagonist is suitably administered serially or in combination with radiological treatments, whether involving irradiation or administration of radioactive substances.

In one embodiment, vascularization of tumors is attacked in combination therapy. The antagonist and one or more other antagonists are administered to tumor-bearing patients at therapeutically effective doses as determined for example by observing necrosis of the tumor or its metastatic foci, if any. This therapy is continued until such time as no further beneficial effect is observed or clinical examination shows no trace of the tumor or any metastatic foci. Then TNF is administered, alone or in combination with an auxiliary agent such as alpha-, beta-, or gamma-interferon, anti-HER2 antibody, heregulin, anti-heregulin antibody, D-factor, interleukin-1 (IL-1), interleukin-2 (IL-2), granulocyte-macrophage colony stimulating factor (GM-CSF), or agents that promote microvascular coagulation in tumors, such as anti-protein C antibody, anti-protein S antibody, or C4b binding protein (see Esmon *et al*., PCT Patent Publication No. WO 91/01753, published 21 February 1991 ), or heat or radiation.

Since the auxiliary agents will vary in their effectiveness it is desirable to compare their impact on the tumor by matrix screening in conventional fashion. The administration of antagonist and TNF is repeated until the desired clinical effect is achieved. Alternatively, the antagonist is administered together with TNF and, optionally, auxiliary agent(s). In instances where solid tumors are found in the limbs or in other locations susceptible to isolation from the general circulation, the therapeutic agents described herein are administered to the isolated tumor or organ. In other embodiments, a FGF or platelet-derived growth factor (PDGF) antagonist, such as an anti-FGF or an anti-POOF neutralizing antibody, is administered to the patient in conjunction with the antagonist. Treatment with antagonist optimally may be suspended during periods of wound healing or desirable vascularization.

In one embodiment, the Eph receptor antagonist is used to treat a vascular anomaly (e.g. small vessel anomalies) resulting from estrogen therapy. Administration of the Eph receptor antagonist may precede or follow administration of estrogen to the patient. Concomitant therapy is also contemplated, for example, where the Eph receptor antagonist and estrogen are provided in the same composition. The dosages of estrogen may correspond to those previously known.

### H. THERAPY WITH EPH RECEPTOR AGONISTS

The present invention provides means for stimulating angiogenesis comprising administering an Eph receptor agonist to a mammal. For example, the agonist may be used to treat conditions associated with the vascular endothelium, such as the treatment of trauma to the vascular network. Examples of such trauma that could be so treated include, but are not limited to, surgical incisions, particularly those involving the heart, wounds, including lacerations, incisions, and penetrations of blood vessels, and surface ulcers involving the vascular endothelium such as diabetic, haemophiliac, and varicose ulcers.

For the traumatic indications referred to above, the Eph receptor agonist will be formulated and dosed in a fashion consistent with good medical practice taking into account the specific disorder to be treated, the condition of the individual patient, the site of delivery of the Eph receptor agonist, the method of administration, and other factors known to practitioners.

Additional indications for the Eph receptor agonist are in the treatment of full-thickness wounds such as dermal ulcers, including the categories of pressure sores, venous ulcers, and diabetic ulcers, as well as of full-thickness burns and injuries where angiogenesis is required to prepare the burn or injured site for a skin graft or flap. In this case the Eph receptor agonist is either applied directly to the site or it is used to soak the skin or flap that is being transplanted prior to grafting. In a similar fashion, the Eph receptor agonist can be used in plastic surgery when reconstruction is required following a burn or other trauma, or for cosmetic purposes.

Angiogenesis is also important in keeping wounds clean and non-infected. The Eph receptor agonist can therefore be used in association with general surgery and following the repair of cuts and lacerations. It is particularly useful in the treatment of abdominal wounds with a high risk of infection. Vascularization is also key to fracture repair, since blood vessels develop at the site of bone injury. Administration of the Eph receptor agonist to the site of a fracture is therefore another utility.

In cases where the Eph receptor agonist is being used for topical wound healing, as described above, it may be administered by any of the routes described below for the re-endothelialization of vascular tissue, or more preferably by topical means. In these cases, it will be administered as either a solution, spray, gel, cream, ointment, or dry powder directly to the site of injury. Slow-release devices directing the Eph receptor agonist to the injured site will also be used. In topical applications, the Eph receptor agonist will be applied at a concentration ranging from about 50 to 1,000 µg/mL, either in a single application, or in dosing regimens that are daily or every few days for a period of one week to several weeks. Generally, the amount of topical formulation administered is that which is sufficient to apply from about 0.1 to 100 µg/cm² of the Eph receptor agonist, based on the surface area of the wound.

The Eph receptor agonist can be used as a post-operative wound healing agent in balloon angioplasty, a procedure in which vascular endothelial cells are removed or damaged, together with compression of atherosclerotic plaques. The Eph receptor agonist can be applied to inner vascular surfaces by systemic or local intravenous application either as intravenous bolus injection or infusions. If desired, the Eph receptor agonist can be administered over time using a micrometering pump. Suitable compositions for intravenous administration comprise the Eph receptor agonist in an amount effective to promote endothelial cell growth and a parenteral carrier material. The Eph receptor agonist can be present in the composition over a wide range of concentrations, for example, from about 50 µg/mL to about 1,000 µg/mL using injections of 3 to 10 mL per patient, administered once or in dosing regimens that allow for multiple applications. Any of the known parenteral carrier vehicles can be used, such as normal saline or 5-10% dextrose.

The Eph receptor agonist can also be used to promote endothelialization in vascular graft surgery. In the case of vascular grafts using either transplanted vessels or synthetic material, for example, the Eph receptor agonist can be applied to the surfaces of the graft and/or at the junctions of the graft and the existing vasculature to promote the growth of vascular endothelial cells. For such applications, the Eph receptor agonist can be applied intravenously as described above for balloon angioplasty or it can be applied directly to the surfaces of the graft and/or the existing vasculature either before or during surgery. In such cases it may be desired to apply the Eph receptor agonist in a thickened carrier material so that it will adhere to the affected surface. Suitable carrier materials include, for example, 1-5% carbopol. The Eph receptor agonist can be present in the carrier over a wide range of concentrations, for example, from about 50 µg/mg to about 1,000 µg/mg. Alternatively, the Eph receptor agonist can be delivered to the site by a micrometering pump as a parenteral solution.

The Eph receptor agonist can also be employed to repair vascular damage following myocardial infarction and to circumvent the need for coronary bypass surgery by stimulating the growth of a collateral circulation. The Eph receptor agonist is administered intravenously for this purpose, either in individual injections or by micrometering pump over a period of time as described above or by direct infusion or injection to the site of damaged cardiac muscle.

The route of Eph receptor agonist administration is in accord with known methods, *e.g.*, those routes set forth above for specific indications, as well as the general routes of injection or infusion by intravenous, intraperitoneal, intracerebral, intramuscular, intraocular, intraarterial, or intralesional means, or sustained release systems as noted below. Eph receptor agonist is administered continuously by infusion or by bolus injection. Generally, where the disorder permits, one should formulate and dose the Eph receptor agonist for site-specific delivery. This is convenient in the case of wounds and ulcers.

An effective amount of Eph receptor agonist to be employed therapeutically will depend, for example, upon the therapeutic objectives, the route of administration, and the condition of the patient. Accordingly, it will be necessary for the therapist to titer the dosage and modify the route of administration as required to obtain the optimal therapeutic effect. Typically, the clinician will administer the Eph receptor agonist until a dosage is reached that achieves the desired effect. A typical daily dosage for systemic treatment might range from about 1 µg/kg to up to 10 mg/kg or more, depending on the factors mentioned above. As an alternative general proposition, the Eph receptor agonist is formulated and delivered to the target site or tissue at a dosage capable of establishing in the tissue an Eph receptor agonist level greater than about 0.1 ng/cc up to a maximum dose that is efficacious but not unduly toxic. This intra-tissue concentration should be maintained if possible by continuous infusion, sustained release, topical application, or injection at empirically determined frequencies. The progress of this therapy is easily monitored by conventional assays.

It is within the scope hereof to combine the Eph receptor agonist therapy with other novel or conventional therapies (e.g., growth factors such as VEGF, acidic or basic fibroblast growth factor (aFGF or bFGF, respectively), platelet-derived growth factor (PDGF), insulin-like growth factor (IGF-I or IGF-II), nerve growth factor (NGF), anabolic steroids, EGF or TGF-β) for enhancing the activity of any of the growth factors, including the Eph receptor agonist, in promoting cell proliferation and repair. It is not necessary that such co-treatment drugs be included *per se* in the compositions of this invention, although this will be convenient where such drugs are proteinaceous. Such admixtures are suitably administered in the same manner and for the same purposes as the Eph receptor agonist used alone. The useful molar ratio of Eph receptor agonist to such secondary growth factors is typically 1:0.1-10, with about equimolar amounts being preferred.

### EXAMPLE 1

This example shows that EphB4 receptor is essential for promoting angiogenic remodeling of primary capillary networks. Mice that lack EphB4 had a more precocious phenotype and died around E9.5-E10. The heart was dilated and major arteries and veins were severely dysmorphic. Most strikingly, angiogenic remodeling of primary extra- and intra-embryonic capillary networks was completely aborted, and large endothelial sheets assembled to vast, poorly structured sinusoids. Thus, the EphB4 pathway governs angiogenesis at an early stage, when the primary vascular system starts to reorganize through sprouting and intussusception.

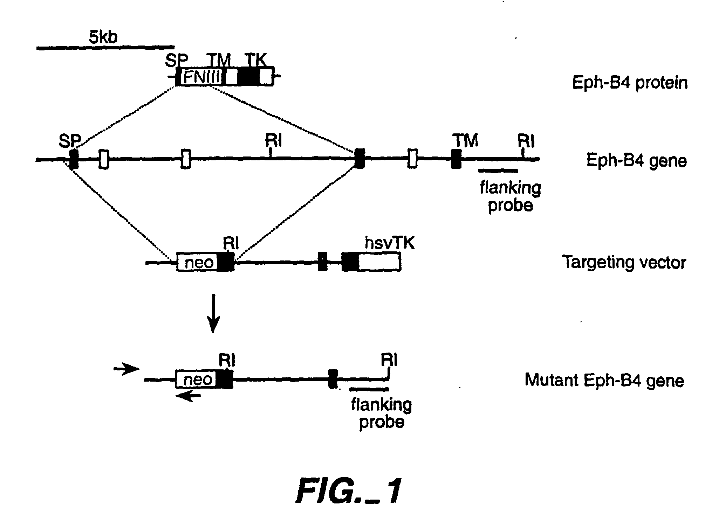

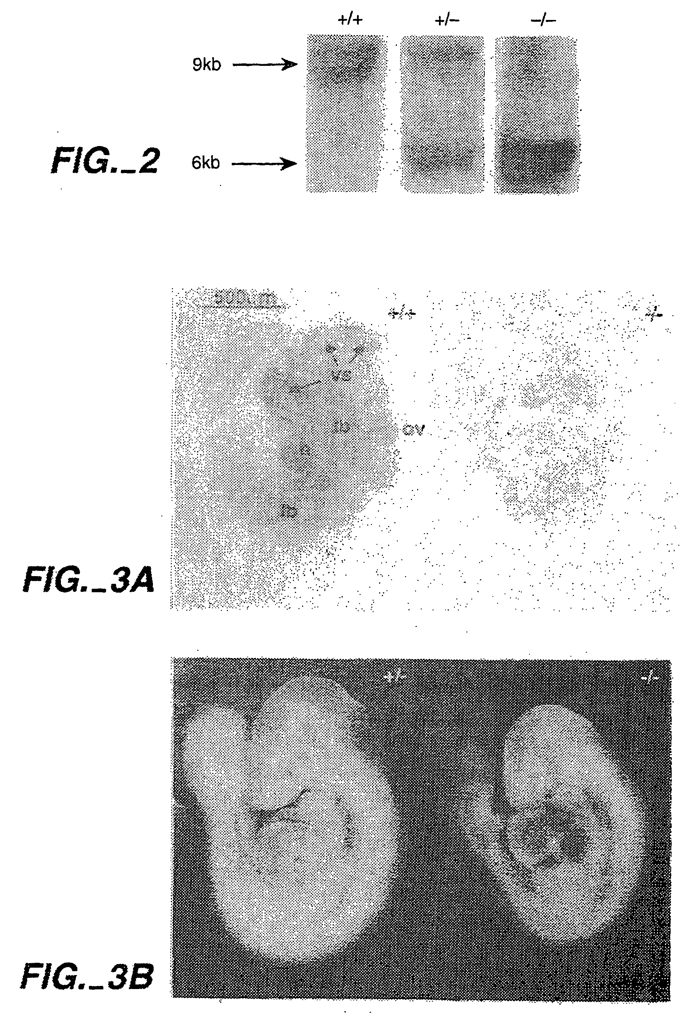

The EphB4 gene was inactivated by transfecting mouse embryonic stem (ES) cells with a replacement vector shown in Fig. 1. Homologous integration occurred with a frequency of approximately I in 200 G-418 and gancyclovir resistant ES cell colonies and resulted in the replacement with the neomycin resistance gene of a region of the EphB4 gene encoding the first 382 amino acid residues of the protein, including the signal peptide. The structure of the mutated EphB4 gene in targeted ES cells and in the progeny of germline chimeras was identified by Southern blot analysis (Fig. 2). Mice heterozygous for the mutation had no apparent phenotypic anomalies and were fertile. However, no EphB4 -/-mice were found among 114 offspring from heterozygous crossings. To determine the time at which the EphB4 mutation was lethal, embryos from F1 intercrosses were examined at different stages. Up to E9.5-E10, viable EphB4 -/- embryos were present in the expected Mendelian ratio. At E10.5 all homozygous mutant embryos were dead as evidenced by extensive apoptosis and/or necrosis.

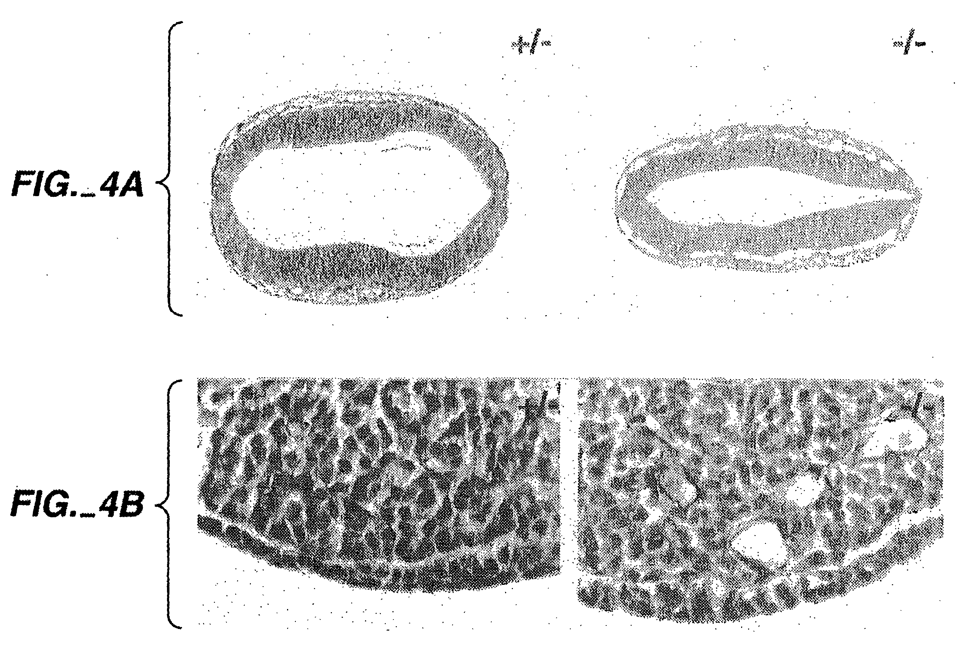

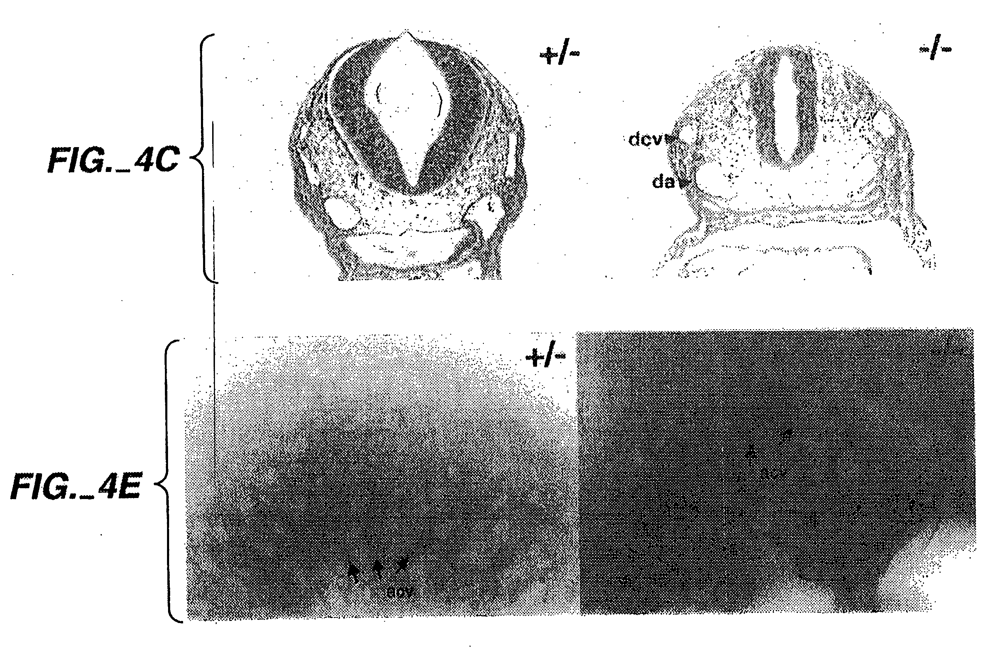

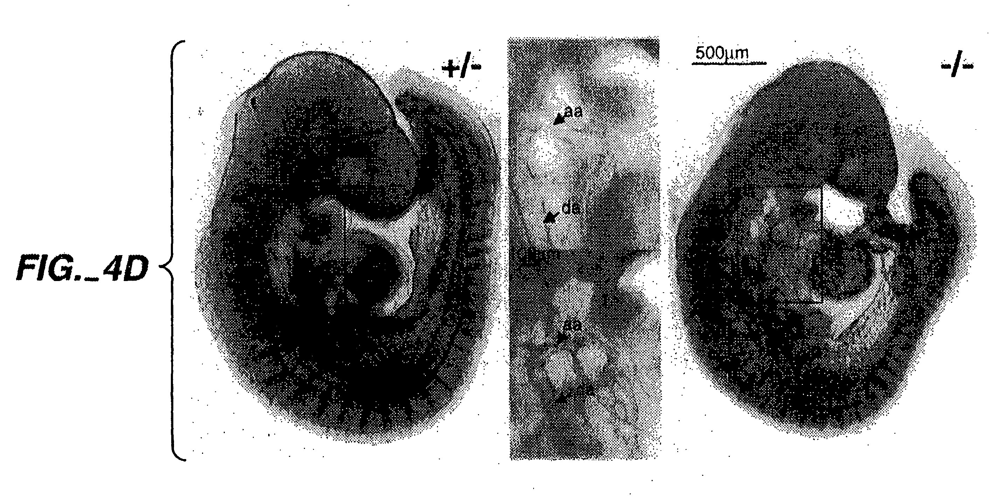

Whole mount *in situ* hybridization in E9.5 embryos revealed specific EphB4 expression predominantly in the ventricular system including the otic vesicles (Fig. 3A). Specific expression was also seen in the developing heart. Otherwise, expression was diffuse and presumably largely mesenchymal, with some preponderance in the limb buds. Mutant embryos were growth retarded and consistently had a dilated heart (Fig. 3B). Histological analysis revealed a striking vascular defect at the level of small vessels. Thus, the capillaries surrounding the neural tube (future pia mater and chorioid plexus) were heavily dilated (Fig. 4A). Immunostaining of platelet endothelial cell adhesion molecule (PECAM) as an endothelial cell marker revealed vast capillary dilations throughout the body, as exemplified by the vessels of the growing limb buds (Fig. 4B). Even though large vessels were normally present and showed no apparent anomalies in tissue sections (Fig. 4C), whole mount staining with anti-PECAM antibodies also revealed severe anomalies of both large arteries and veins (Fig. 4D & 4E). These vessels were generally normal in size, but dysmorphic and irregularly shaped. Strikingly, the branching and merging of collecting vessels was disorganized as exemplified by the cardinal head veins (Fig. 4E).

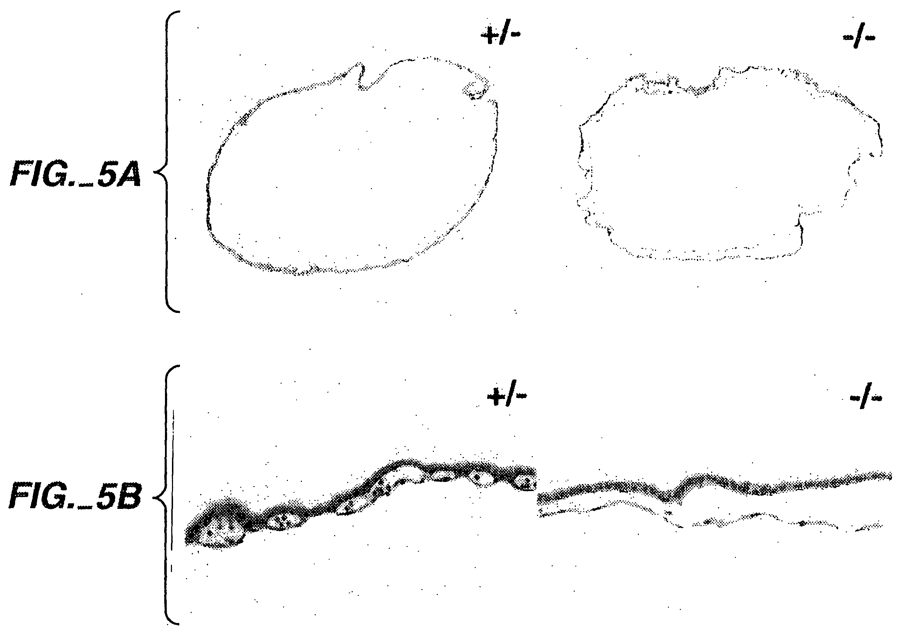

The vascular defect was most dramatic in the yolk sac which consisted of large endothelial sinusoids instead of the primary capillary network that normally compartmentalizes blood islands (Fig. 5A). Large vessels were undetectable, with exception of the vitelline artery and vein. The endothelial layer that lines these sinusoids and covers the yolk sac epithelium showed no apparent anomalies when examined both histologically and by electron microscopy, and endothelial cells were present at normal density (Fig. 5B). Red blood cell development was normal as evidenced by benzidine staining of hemoglobin, and mutant E9.5 embryos had a functional cardio-vascular system, since blood cells were observed throughout the vascular system. The presumably low peripheral vascular resistance may, however, have caused lethal hemodynamic insufficiency.

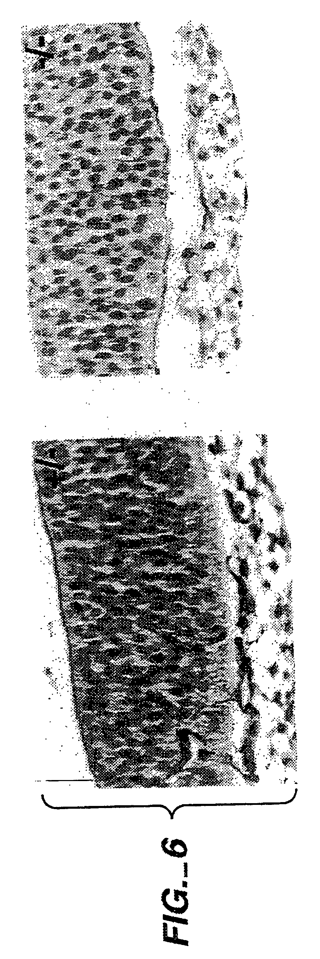

Thus, early stages of vascular development including the differentiation of hematopoietic stem cells and angioblasts from hemangioblast precursors, the migration of angioblasts to remote sites such as the perineural vascular plexus, and the formation of primary capillary networks remained unaffected by the inactivation of the EphB4 gene. The pathological endothelial sinusoids that formed throughout the body of mutant E9.5 embryos, where capillary beds normally start to vascularize peripheral tissues, therefore reflect a severe defect in the process angiogenic remodeling which underlies the formation of mature capillary networks. These are believed to form through an interplay of sprouting of new, and splitting or intussusception of preexisting vessels. The defect observed most strikingly in the yolk sac of E9.5 EphB4 -/embryos might reflect an arrest at a stage at which primary vessels merge before vascular reorganization is initiated through transluminal partitioning. Sprouting angiogenesis, which occurs for example in response to VEGF produced in the periventricular layer of the neural tube, may also be affected in EphB4 -/- embryos, since the neuroepithelium of mutant embryos failed to become vascularized (Fig. 6). Indeed, normally differentiated endothelial cells seem to arrest at the interface between mesenchyme and neuroepithelium and form a continuous endothelial sheet instead of invading the epithelial layer where at this stage EphB4 expression is most pronounced (Fig. 3A).

### Methods

*Targeted disruption of the EphB4 gene:* An 18 kb gene fragment encoding the entire extracellu lar domain and a portion of the intracellular domain of murine EphB4 was isolated from a lambda DashII strain 129Sv library (Stratagene) using a murine EphB4 cDNA probe [Andres *et al. Oncogene* 9: 1461-1467 (1994)] and subcloned into a B luescript vector as 4 XbaI fragments. A replacement vector was constructed by inserting a 1.1 Kb BspMII-ApaI fragment containing sequences upstream of the translation initiation codon into the NotI site of pTK.neo.ums [Reis *et al. EMBO J.* 13: 4798-4806 ( 1994)], and a 4.0 kb fragment Xhol-XbaI fragment containing the exons encoding for amino acids 383-563 of the immature protein into the ClaI of pTK.neo.ums, adjacent to the polyoma enhancer promoter-driven herpes simplex virus thymidine kinase gene. The targeting construct replaced 11kb of the EphB4 gene with the neomycin resistance gene placed under the control of the PGK promoter, thereby deleting the gene portion encoding the first 382 amino acids of the EphB4 protein. ES cells [Reis *et al. EMBO J.* 13: 4798-4806 (1994)] were electroporated and colonies selected as described [Spencer *et al. J. Exp. Med.* 187: 571-578 (1998)]. Homologous recombination was detected by PCR using an EphB4 specific sense primer corresponding to a genomic sequence immediately upstream to the 5' end of the targeting vector (pHTKc1, ACTTAACGTGCACGCACTGAT - SEQ ID NO:1) and an anti-sense primer specific for the neomycin resistance gene (pHTKc2, ACTGAAGCGGGAAGGGACTGG - SEQ ID NO:2). Homologous integration was confirmed by Southern blot analysis of EcoRI digested genomic DNA, using a ³²P labeled 1.5 Kb XbaI-EcoRI probe derived from genomic sequences downstream of the targeting vector. Positive clones were identified by the presence of a 6 kb EcoRI fragment in addition to the 9 kb EcoRI fragment typical of the EphB4 wild-type allele.

*Embryological analysis*: Genotypes were determined from yolk-sac or embryonic DNA using primer pairs specific for the neomycin resistance gene (pNeo 474+, GCCGCGCTGTTCTCCTCTTCC - SEQ ID NO:3 - and pNeo 1513-, TCCCAATCCTCCCCCTTGCTGT - SEQ ID NO:4), or encompassing the deletion within the EphB4 gene (pHtkV7-2 152+, CTTTATGTGAGGYTGGGGACA - SEQ ID NO:5 and pHtk p469- wt, CCGTATCACGTTCGCTCTCGT - SEQ ID NO:6).

Embryos removed between E8.5 to E11.5 were fixed in 4% paraformaldehyde for 2h at room temperature. For histological analysis embryos and yolk-sac were dehydrated, paraffin embedded, sectioned (generally 8µm) and counterstained with Gill's hematoxylin according to standard protocols.

*Whole mount in situ hybridization*: Whole mount *in situ* hybridization was performed as described [Wilkinson, D.G. "Whole mount in situ hybridization of vertebrate embryos". in *In Situ Hybridization: A Pratical Approach* ed. IRL Press, O., ( 1992) pps. 75-83]. An EphB4 anti-sense probe was synthesized from a SstI EphB4 cDNA fragment (nucleotides 2 - 644 of the EphB4 open reading frame) that was subcloned into pBlKSII+ (Stratagene).

*Immunohistochemistry*: Whole-mount immunohistochemistry was performed essentially as described [Schlaeger *et al. Development* 121: 1089-1098 (1995)]. In brief, paraformaldehyde fixed embryos and yolk-sacs were rinsed in PBS, dehydrated in methanol, bleached in 5% hydrogen peroxide in methanol for 4-5 hours at room temperature and rinsed with methanol. Samples were rehydrated, incubated for 1 hour at 4°C in PBS containing 0.1 % Triton-X-100 (PBST) and 10% FCS, and overnight at 4°C with an anti-PECAM Mab (Pharmingen, clone MEC13.3, 1/250 dilution). Subsequently, samples were washed five times for 1 hour at 4C° in PBST, incubated overnight at 4°C with horseradish peroxidase-conjugated goat anti-rat IgG (TACO Immunologicals #6470, 1/100 dilution), washed five times for 1 hour at 4°C in PBST and once for 20 minutes at room temperature in PBST supplemented with 0.2% BSA (PBT). Peroxidase staining was performed by incubation for 20 minutes in 0.3 mg/ml DAB (Sigma) in PBT followed by the addition of hydrogen peroxydase to the final concentration of 0.03%. After 10 minutes the reaction was stopped by rinsing in PBT and PBST. Stained embryos were post-fixed in 4% paraformaldehyde.

## Claims

1. Use of an Eph receptor antagonist, as an active ingredient, for the manufacture of a composition for the treatment of cancer in a mammal.

2. Use according to claim 1 wherein the mammal has a solid malignant tumour.

3. Use of an Eph receptor antagonist, as an active ingredient, for the manufacture of a composition for the treatment of cancer or a related condition in a mammal, wherein the cancer or related condition is a breast carcinoma, lung carcinoma, gastric carcinoma, esophageal carcinoma, colorectal carcinoma, liver carcinoma, ovarian carcinoma, thecoma, arrhenoblastoma, cervical carcinoma, endometrial carcinoma, endometrial hyperplasia, endometriosis, fibrosarcoma, choriocarcinoma, head or neck cancer, nasopharyngeal carcinoma, laryngeal carcinoma, hepatoblastoma, Kaposi's sarcoma, melanoma, skin carcinoma, hemagioma, cavernous carcinoma, hemagioblastoma, pancreas carcinoma, retinoblastoma, astrocytoma, glioblastoma, Schwannoma, oligodendroglioma, medulloblastoma, neuroblastoma, rhabdomyosarcoma, osteogenic carcinoma, leiomyosarcoma, urinary tract carcinoma, thyroid carcinoma, Wilm's tumour, renal cell carcinoma, prostate carcinoma, abnormal vascular proliferation associated with phakomatoses, edema, or Meig's syndrome.

4. Use according to any one of the preceding claims further comprising the use of a further therapeutic agent in the manufacture of the same or different composition.

5. Use according to claim 4 wherein the further therapeutic agent is a tumour necrosis factor (TNF); or an antagonist of an acidic or basic fibroblast growth factor (FGF), hepatocyte growth factor (HGF), tissue factor (TF), protein C, protein S, platelet-derived growth factor (PDGF), or HER 2 receptor.

6. Use of an Eph receptor antagonist for the manufacture of a composition for the treatment of a non-neoplastic condition in a mammal, wherein said non-neoplastic condition is rheumatoid arthritis, psoriasis, atherosclerosis, diabetic or other proliferative retinopathy, retrolental fibroplasia, neovascular glaucoma, age-related macular degeneration, thyroid hyperplasia, corneal or other tissue transplantation, chronic inflammation, lung inflammation, nephritic syndrome, preeclampsia, ascites, pericardial effusion, pleural effusion, or a vascular anomaly resulting from estrogen therapy.

7. Use according to any one of claims 1 to 6 wherein the antagonist binds an Eph receptor.

8. Use according to claim 7 wherein the Eph receptor is EphB4.

9. Use according to claim 7 wherein the Eph receptor is EphB2.

10. Use according to any one of claims 1 to 6 wherein the antagonist binds an ephrin ligand.

11. Use according to claim 10 wherein the ephrin ligand is Ephrin-B2.

12. Use according to any one of claims 1 to 11 wherein the antagonist comprises an antibody.

13. Use according to any one of claims 1 to 11 wherein the antagonist comprises an immunoadhesin.

14. Use according to claim 10 or claim 11 wherein the antagonist comprises a portion of the Eph receptor which binds the Eph ligand but lacks a domain conferring biological activity.

15. Use of an Eph receptor agonist for the manufacture of a composition for the treatment of a trauma to the vascular network in a mammal.

16. Use according to claim 15 wherein the trauma is a surgical incision; wound; laceration, incision or penetration of blood vessels; or a surface ulcer involving the vascular endothelium.

17. Use according to claim 16 wherein the surface ulcer is a diabetic, haemophiliac or varicose ulcer.

18. Use according to claim 15 wherein the trauma is vascular graft surgery, balloon angioplasty or myocardial infarction.

19. Use of an Eph receptor agonist for the manufacture of a composition for the treatment of a full-thickness wound in a mammal.

20. Use according to claim 19 wherein the full-thickness wound is a dermal ulcer, full-thickness burn or injury.

21. Use according to any one of claims 15 to 20 wherein the agonist binds an Eph receptor.

22. Use according to claim 21 wherein the Eph receptor is EphB4.

23. Use according to claim 21 wherein the Eph receptor is EphB2.

24. Use according to any one of claims 15 to 20 wherein the agonist binds an ephrin ligand.

25. Use according to claim 24 wherein the ephrin ligand is Ephrin-B2.

26. Use according to any one of claims 15 to 25 wherein the agonist comprises an antibody.

27. Use according to claims 12 or 26 wherein the antibody is an antibody fragment.

28. Use according to claim 27 wherein the antibody fragment is a Fab, Fab', F(ab')₂ or Fv fragment.

29. Use according to any one of the preceding claims wherein the mammal is a human.

## Patentansprüche

1. Verwendung eines Eph-Rezeptorantagonisten als Wirkstoff zur Herstellung einer Zusammensetzung zur Behandlung von Krebs in einem Säugetier.

2. Verwendung nach Anspruch 1, worin das Säugetier einen festen bösartigen Tumor aufweist.

3. Verwendung eines Eph-Rezeptorantagonisten als Wirkstoff zur Herstellung einer Zusammensetzung zur Behandlung von Krebs oder eines verwandten Leidens in einem Säugetier, worin der Krebs oder das verwandte Leiden ein(e) Brustkarzinom, Lungenkarzinom, Magenkarzinom, Ösophaguskarzinom, Kolorektalkarzinom, Leberkarzinom, Ovarialkarzinom, Thekazellentumor, Arrhenoblastom, Zervixkarzinom, Endometriumkarzinom, Endometriumhyperplasie, Endometriose, Fibrosarkom, Chorionkarzinom, Kopf- oder Halskrebs, Nasopharynxkarzinom, Larynxkarzinom, Hepatoblastom, Kaposi-Sarkom, Melanom, Hautkarzinom, Hämangiom, Kavernom, Hämangioblastom, Pankreaskarzinom, Retinoblastom, Astrozytom, Glioblastom, Schwannom, Oligodendrogliom, Medulloblastom, Neuroblastom, Rhabdomyosarkom, osteogenes Karzinom, Leiomyosarkom, Harnwegskarzinom, Schilddrüsenkarzinom, Wilms-Tumor, Nierenzellkarzinom, Prostatakarzinom, anomale Gefäßwucherung in Zusammenhang mit Phakomatose, Ödem oder Meigs-Syndrom ist.

4. Verwendung nach einem der vorangegangenen Ansprüche, außerdem die Verwendung eines weiteren Therapeutikums bei der Herstellung der gleichen oder einer anderen Zusammensetzung umfassend.

5. Verwendung nach Anspruch 4, worin das weitere Therapeutikum ein Tumor-Nekrose-Faktor (TNF); oder ein Antagonist eines sauren oder basischen Fibroblastenwachstumsfaktors (FGF), eines Hepatozytenwachstumsfaktors (HGF), Gewebefaktors (TF), Proteins C, Proteins S, aus Blutplättchen gewonnenen Wachstumsfaktors (PDGF) oder HER-2-Rezeptors ist.

6. Verwendung eines Eph-Rezeptorantagonisten zur Herstellung einer Zusammensetzung zur Behandlung eines nicht neoplastischen Leidens in einem Säugetier, worin das nicht neoplastische Leiden rheumatoide Arthritis, Psoriasis, Atherosklerose, Diabetes oder andere proliferative Retinopathie, retrolentale Fibroplasie, Neovaskularisationsglaukom, altersbedingte Makuladegeneration, Schilddrüsenüberfunktion, Hornhaut- oder andere Gewebetransplantation, chronische Entzündung, Lungenentzündung, nephritisches Syndrom, Präeklampsie, Aszites, Perikarderguss, Pleuraerguss oder Gefäßanomalie aufgrund einer Östrogentherapie ist.

7. Verwendung nach einem der Ansprüche 1 bis 6, worin der Antagonist einen Eph-Rezeptor bindet.

8. Verwendung nach Anspruch 7, worin der Eph-Rezeptor EphB4 ist.

9. Verwendung nach Anspruch 7, worin der Eph-Rezeptor EphB2 ist.

10. Verwendung nach einem der Ansprüche 1 bis 6, worin der Antagonist einen Ephrin-Liganden bindet.

11. Verwendung nach Anspruch 10, worin der Ephrin-Ligand Ephrin-B2 ist.

12. Verwendung nach einem der Ansprüche 1 bis 11, worin der Antagonist einen Antikörper umfasst.

13. Verwendung nach einem der Ansprüche 1 bis 11, worin der Antagonist ein Immunoadhäsin umfasst.

14. Verwendung nach Anspruch 10 oder Anspruch 11, worin der Antagonist einen Teil des Eph-Rezeptors umfasst, der den Eph-Liganden bindet, aber keine Domäne aufweist, die biologische Aktivität verleiht.

15. Verwendung eines Eph-Rezeptoragonisten zur Herstellung einer Zusammensetzung zur Behandlung eines Traumas am Gefäßnetz eines Säugetiers.

16. Verwendung nach Anspruch 15, worin das Trauma ein(e) chirurgische Inzision; Wunde; Lazeration, Inzision oder Penetration von Blutgefäßen; oder das vaskuläre Endothel umfassende Oberflächengeschwür ist.

17. Verwendung nach Anspruch 16, worin das Oberflächengeschwür ein diabetisches, hämophiles oder variköses Geschwür ist.

18. Verwendung nach Anspruch 15, worin das Trauma Gefäßtransplantation, Ballondilatation oder Myokardinfarkt ist.

19. Verwendung eines Eph-Rezeptoragonisten zur Herstellung einer Zusammensetzung zur Behandlung einer "Full-Thickness"-Wunde an einem Säugetier.

20. Verwendung nach Anspruch 19, worin die "Full-Thickness"-Wunde ein Hautgeschwür, eine "Full-Thickness"-Verbrennung oder -Verletzung ist.

21. Verwendung nach einem der Ansprüche 15 bis 20, worin der Agonist einen Eph-Rezeptor bindet.

22. Verwendung nach Anspruch 21, worin der Eph-Rezeptor EphB4 ist.

23. Verwendung nach Anspruch 21, worin der Eph-Rezeptor EphB2 ist.

24. Verwendung nach einem der Ansprüche 15 bis 20, worin der Agonist einen Ephrin-Liganden bindet.

25. Verwendung nach Anspruch 24, worin der Ephrin-Ligand Ephrin-B2 ist.

26. Verwendung nach einem der Ansprüche 15 bis 25, worin der Agonist einen Antikörper umfasst.

27. Verwendung nach einem der Ansprüche 12 oder 26, worin der Antikörper ein Antikörperfragment ist.

28. Verwendung nach Anspruch 27, worin das Antikörperfragment ein Fab-, Fab'-, F(ab')₂- oder Fv-Fragment ist.

29. Verwendung nach einem der vorangegangenen Ansprüche, worin das Säugetier ein Mensch ist.

## Revendications

1. Utilisation d'un antagoniste de récepteur Eph, comme ingrédient actif, pour la préparation d'une composition pour le traitement du cancer chez un mammifère.

2. Utilisation selon la revendication 1, où le mammifère a une tumeur maligne solide.

3. Utilisation d'un antagoniste de récepteur Eph, comme ingrédient actif, pour la préparation d'une composition pour le traitement du cancer ou d'un état apparenté chez un mammifère, où le cancer ou l'état apparenté est les carcinomes du sein, les carcinomes du poumon, les carcinomes gastriques, les carcinomes de l'oesophage, les carcinomes colorectaux, les carcinomes hépatiques, les carcinomes ovariens, les thécomes, les arrhénoblastomes, les carcinomes du col, les carcinomes de l'endomètre, l'hyperplasie endométriale, l'endométriose, les fibrosarcomes, les choriocarcinomes, le cancer de la tête et du cou, le carcinome nasopharyngien, les carcinomes du larynx, les hépatoblastomes, le sarcome de Kaposi, le mélanome, les carcinomes de la peau, l'hémangiome, l'hémangiome caverneux, l'hémangioblastome, les carcinomes du pancréas, le rétinoblastome, l'astrocytome, le glioblastome, le Schwannome, l'oligodendrogliome, le médulloblastome, les neuroblastomes, le rhabdosarcome, le sarcome ostéogène, les leiomyosarcomes, les carcinomes du tractus urinaire, les carcinomes de la thyroïde, la tumeur de Wilm, le carcinome des cellules rénales, le carcinome de la prostate, une prolifération vasculaire anormale associée à des phacomatoses, des oedèmes ou le syndrome de Meig.

4. Utilisation selon l'une quelconque des revendications précédentes, comprenant en outre, l'utilisation d'un autre agent thérapeutique dans la préparation d'une même composition ou d'une autre composition.

5. Utilisation selon la revendication 4, où l'autre agent thérapeutique est un facteur de nécrose tumorale (TNF), ou un antagoniste d'un facteur de croissance des fibroblastes acide ou basique (FGF), du facteur de croissance des hépatocytes (HGF), du facteur tissulaire (TF), de la protéine C, de la protéine S, du facteur de croissance dérivé des plaquettes (PDGF) ou du récepteur HER 2.

6. Utilisation d'un antagoniste de récepteur Eph, pour la préparation d'une composition pour le traitement d'un état non néoplasique chez un mammifère, où ledit état non néoplasique est la polyarthrite rhumatoïde, le psoriasis, l'athérosclérose, la réthinopathie proliférative diabétique ou autre, la fibroplasie rétrolentale, le glaucome néovasculaire, la dégénérescence maculaire reliée à l'âge, l'hyperplasie thyroïdienne, une transplantation cornéenne ou d'un autre tissu, l'inflammation chronique, l'inflammation pulmonaire, le syndrome néphrétique, la prééclampsie, les ascites, l'épanchement péricardique, l'épanchement pleural ou une anomalie vasculaire résultant d'une thérapie à l'oestrogène.

7. Utilisation selon l'une quelconque des revendications 1 à 6, où l'antagoniste se lie à un récepteur Eph.

8. Utilisation selon la revendication 7, où le récepteur Eph est EphB4.

9. Utilisation selon la revendication 7, où le récepteur Eph est EphB2.

10. Utilisation selon l'une quelconque des revendications 1 à 6, où l'antagoniste se lie à un ligand éphrine.

11. Utilisation selon la revendication 10, où le ligand éphrine est l'éphrine-B2.

12. Utilisation selon l'une quelconque des revendications 1 à 11, où l'antagoniste comprend un anticorps.

13. Utilisation selon l'une quelconque des revendications 1 à 11, où l'antagoniste comprend une immunoadhésine.

14. Utilisation selon la revendication 10 ou 11, où l'antagoniste comprend une partie du récepteur Eph, qui lie le ligand Eph, mais qui ne présente pas de domaine conférant une activité biologique.

15. Utilisation d'un agoniste de récepteur Eph, pour la préparation d'une composition pour le traitement d'un traumatisme du réseau vasculaire chez un mammifère.

16. Utilisation selon la revendication 15, où le traumatisme est une incision chirurgicale ; une lésion ; une lacération, une incision ou une pénétration de vaisseaux sanguins ; ou un ulcère de surface impliquant l'endothélium vasculaire.

17. Utilisation selon la revendication 16, où l'ulcère de surface est un ulcère diabétique, hémophilique ou variqueux.

18. Utilisation selon la revendication 15, où le traumatisme est une chirurgie de greffe vasculaire, une angioplastie par ballonnet ou l'infarctus du myocarde.

19. Utilisation d'un agoniste de récepteur Eph, pour la préparation d'une composition pour le traitement d'un lésion profonde chez un mammifère.

20. Utilisation selon la revendication 19, où la lésion profonde est un ulcère dermique, une brûlure ou une blessure profonde.

21. Utilisation selon l'une quelconque des revendications 15 à 20, où l'agoniste se lie au récepteur Eph.

22. Utilisation selon la revendication 21, où le récepteur Eph est EphB4.

23. Utilisation selon la revendication 21, où le récepteur Eph est EphB2.

24. Utilisation selon l'une quelconque des revendications 15 à 20, où l'agoniste se lie au ligand éphrine.

25. Utilisation selon la revendication 24, où le ligand éphrine est l'éphrine-B2.

26. Utilisation selon l'une quelconque des revendications 15 à 25, où l'agoniste comprend un anticorps.

27. Utilisation selon les revendications 12 ou 26, où l'anticorps est un fragment d'anticorps.

28. Utilisation selon la revendication 27, où le fragment d'anticorps est un fragment Fab, Fab', F(ab')₂ ou Fv.

29. Utilisation selon l'une quelconque des revendications précédentes, où le mammifère est un humain.
